(19) Europäisches Patentamt
European Patent Office
Office européen des brevets

(11) EP 2 066 297 B1

(12) **EUROPEAN PATENT SPECIFICATION**

(45) Date of publication and mention of the grant of the patent:
**11.05.2011 Bulletin 2011/19**

(21) Application number: **07838832.9**

(22) Date of filing: **25.09.2007**

(51) Int Cl.:
***A61K 8/87*** *(2006.01)* ***A61K 8/04*** *(2006.01)*
***A61Q 19/00*** *(2006.01)* ***A61Q 1/06*** *(2006.01)*

(86) International application number:
**PCT/US2007/020703**

(87) International publication number:
**WO 2008/039466 (03.04.2008 Gazette 2008/14)**

(54) **COSMETIC COMPOSITIONS**

KOSMETISCHE ZUSAMMENSETZUNGEN

COMPOSITIONS COSMÉTIQUES

(84) Designated Contracting States:
**DE ES FR GB IT NL**

(30) Priority: **25.09.2006 US 846991 P**

(43) Date of publication of application:
**10.06.2009 Bulletin 2009/24**

(73) Proprietor: **E.I. DU PONT DE NEMOURS AND COMPANY**
**Wilmington, DE 19898 (US)**

(72) Inventor: **SUNKARA, Hari Babu**
**Hockessin DE 19707 (US)**

(74) Representative: **Hirsch, Marc-Roger**
**Hirsch & Associés**
**58, Avenue Marceau**
**75008 Paris (FR)**

(56) References cited:
**WO-A-2008/013924**
**US-A- 5 972 354**
**US-A1- 2004 141 942**
**US-A1- 2004 225 107**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

This invention relates to cosmetic compositions containing aqueous dispersions of nano-sized polyurethane ionomers based on polytrimethylene ether glycol ("PO3G"). In a particularly preferred embodiment, the PO3G is derived predominantly from monomers obtained from renewable resources, making the cosmetic compositions of this embodiment of the present invention more environmentally friendly in terms of their manufacture, use and disposal.

### BACKGROUND OF THE INVENTION

There are a large number of cosmetic products available, most of which are in the form of solutions, creams, ointments, lotions, gels, emulsions or solids. There are a variety of uses for these cosmetic products including, e.g., skin care, bath and body care, deodorants, hand and foot care, facial care, hair care, shaving products, dental care, toiletry and personal lubrication. The ingredients in the formulated products in general serve as emollients, humectants, moisturizers, emulsifiers, lubricants, antimicrobials, cosmetics, fragrances, rheology modifiers, etc. Some of the products are solvent-based and others are water-based.

Most often cosmetic products contain an active ingredient incorporated in a delivery vehicle. The desired effect of a cosmetic product is achieved either by the cosmetic active ingredients or by the vehicle itself at the site of application, in most cases on the skin, hair and/or nails.

The major types of cosmetic vehicles most frequently fall into the following categories: (a) solutions; (b) emulsions, both oil-in-water and water-in-oil, including (for example) lotions and creams; (c) suspensions; (d) gels; and (e) solids (including semi-solids) including (for example) stick products. An extensive discussion of personal care and cosmetic vehicles is found in Handbook of Cosmetic Science and Technology, Second Edition, edited by M Paye, A. O. Barel and H. I. Maibach, pages 99-123 (2005).

Cosmetic products based on aqueous polyurethane dispersions are in a general sense known in the art. See, for example, US5874072, US5968494, US5972354, US2002/0034558A1 and US2004/0141942A1.

The majority of ingredients used in these personal care products are synthetic and are derived from petrochemical sources. The recent trend of the industry is to provide products to consumers that are natural and reduced in petroleum-based product content.

Aqueous dispersions of polyurethane ionomers based in part on ingredients derived from renewable resources are disclosed in commonly owned U.S. Application Ser. No. 11/782098 (filed 24 July 2007, and entitled "Polytrimethylene Ether-Based Polyurethane lonomers"). The use of these aqueous dispersions in personal care applications is mentioned but not exemplified.

There is a need for all manufactures to provide products reduced environmental impacts. There is also an environmental advantage for manufacturers to provide products of renewably based sources. There thus exists a need for cosmetic products comprised of ingredients not derived from petroleum but from renewable resources. In addition, there is a need for ingredients and products that are environmentally friendly in respect to their manufacturing processes, their uses and their disposal.

### SUMMARY OF THE INVENTION

In a first aspect, the present invention relates to a cosmetic composition comprising an aqueous continuous phase having dispersed therein a nano-sized water-dispersible polyurethane and at least one cosmetic additive, wherein the water-dispersible polyurethane is a polyurethane ionomer comprising a polymeric backbone having ionic and/or ionizable functionality incorporated into, pendant from and/or terminating said polymeric backbone, wherein the polymeric backbone comprises one or more non-ionic segments derived from a reaction product of polytrimethylene ether glycol ("PO3G") and a polyisocyanate. Preferably, the polyurethane ionomer has sufficient ionic functionality in order to render the polyurethane dispersible in the aqueous continuous phase. Preferably, all of the ingredients (polyurethane, active cosmetic ingredient and optional additives) are substantially evenly and stably dispersed throughout the aqueous continuous phase, and/or can be readily substantially evenly redispersed, for example, by low energy mixing (such as hand shaking).

In a second aspect, the present invention relates to a cosmetic composition comprising a solid or semi-solid continuous phase having dispersed therein an aqueous vehicle, a nano-sized water-dispersible polyurethane and at least one cosmetic additive, wherein the water-dispersible polyurethane is a polyurethane ionomer comprising a polymeric backbone having ionic and/or ionizable functionality incorporated into, pendant from and/or terminating said polymeric backbone, wherein the polymeric backbone comprises one or more non-ionic segments derived from a reaction product of PO3G and a polyisocyanate. Preferably, the polyurethane ionomer has sufficient ionic functionality in order to render

the polyurethane dispersible in the aqueous vehicle. Preferably, all of the ingredients (aqueous vehicle, polyurethane, active cosmetic ingredient and optional additives) are substantially evenly and stably dispersed throughout the continuous phase.

All ingredients of the compositions in accordance with the present invention should be pharmacologically acceptable.

In a preferred embodiment, the polyurethane is a film-forming polyurethane.

In a particularly preferred embodiment of the present invention, the P03G used in the polyurethane is produced predominantly from monomers (such as 1,3-propane diol) that are biologically-derived.

The cosmetic compositions in accordance with the first aspect of the present invention are typically prepared by mixing the active cosmetic ingredient (and other optional additives) into an aqueous dispersion of a nanoparticle-sized polyurethane ionomer. In a preferred embodiment, when the active cosmetic ingredient is neither soluble nor dispersble in the continuous phase, the polyurethane ionomer acts as a dispersing/emulsifying agent for the ingredient.

The cosmetic compositions in accordance with the second aspect of the present invention are typically prepared by mixing the active cosmetic ingredient (and other optional additives) into an aqueous dispersion of the nanoparticle-sized polyurethane ionomer, then adding a solidifying agent (such as a gelling agent) to result in a solid or semi-sold product.

Cosmetic compositions in accordance with the first aspect are flowable at ambient temperatures (such as 25°C) and include, for example, brushable lip gloss products, hair and skin lotions, shampoos, nail polishes, hair care styling products (such as intended for aerosol application) and the like.

Cosmetic compositions in accordance with the second aspect include, for example, lipsticks, gels, deodorant sticks and the like. While not intended to be flowable at ambient temperatures (such as 25°C), these compositions may become flowable/spreadable upon application to a warmer surface such as skin, lips and nails.

These and other features and advantages of the present invention will be more readily understood by those of ordinary skill in the art from a reading of the following detailed description.

## DETAILED DESCRIPTION OF THE PREFERRED EMBODIMENTS

Unless otherwise defined, all technical and scientific terms used herein have the same meaning as commonly understood by one of ordinary skill in the art to which this invention belongs. In case of conflict, the present specification, including definitions, will control.

Except where expressly noted, trademarks are shown in upper case.

Unless stated otherwise, all percentages, parts, ratios, etc., are by weight.

When an amount, concentration, or other value or parameter is given as either a range, preferred range or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

When the term "about" is used in describing a value or an end-point of a range, the disclosure should be understood to include the specific value or end-point referred to.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Use of "a" or "an" are employed to describe elements and components of the invention. This is done merely for convenience and to give a general sense of the invention. This description should be read to include one or at least one and the singular also includes the plural unless it is obvious that it is meant otherwise.

The materials, methods, and examples herein are illustrative only and, except as specifically stated, are not intended to be limiting. Although methods and materials similar or equivalent to those described herein can be used in the practice or testing of the present invention, suitable methods and materials are described herein.

The present invention is directed to cosmetics compositions that are derived from aqueous dispersions of polyurethane ionomers such as disclosed in previously incorporated U.S. Application Ser. No. 11/782098 (filed 24 July 2007, and entitled "Polytrimethylene Ether-Based Polyurethane Ionomers"). Particularly, the aqueous dispersions are of nanoparticle-sized polyurethane ionomers.

"Nanoparticle-sized" as used herein refers to particles of less than 1 micron. Preferably, the particle size is

between about 0.005 to about 0.5 micron. The average particle size preferably should be less than about 0.5 micron, and preferably between about 0.005 to about 0.3 micron. The small particle size enhances the stability of the dispersed particles. As used herein, particle size refers to a number average particle size as measured by conventional particle size measuring techniques well known to those skilled in the art, such as sedimentation field flow fractionation, photon correlation spectroscopy (PCS), or disk centrifugation. When PCS is used as the method of particle sizing, the average particle diameter is the Z-average particle diameter known to those skilled in the art.

The cosmetic compositions in accordance with the present invention are either flowable (dispersions/emulsions/suspensions) or solid/semi-solid, containing at least one active cosmetic ingredient, a nanoparticle-sized polyurethane ionomer and an aqueous vehicle. The physical form of the cosmetic composition will determine how these various components are contained in the composition.

Polyurethane Ionomer

As indicated previously, the polyurethane ionomer comprises a polymeric backbone having ionic and/or ionizable functionality incorporated into, pendant from and/or terminating said polymeric backbone, wherein the polymeric backbone comprises one or more non-ionic segments derived from a reaction product of PO3G and a polyisocyanate, and wherein said polyurethane ionomer preferably has sufficient ionic functionality in order to render the polyurethane dispersible in the aqueous continuous phase (aqueous vehicle).

Preferably, at least about 20 wt%, more preferably at least about 25 wt%, and still more preferably at least about 40 wt%, of the polyurethane (based on the weight of the polyurethane) comprises one or more non-ionic segments of the general formula (I):

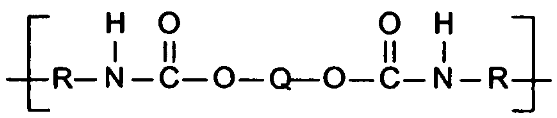 (I)

wherein:

each R individually is the residue of a diisocyanate compound after abstraction of the isocyanate groups; and

Q is the residue of an oligomeric or polymeric diol after abstraction of the hydroxyl groups, wherein the oligomeric or polymeric diol is PO3G.

Preferably, Q in and of itself constitutes at least about 20 wt%, more preferably at least about 25 wt%, and still more preferably at least about 40 wt%, of the polyurethane, based on the weight of the polyurethane.

The polyurethane is preferably prepared from (a) a polyol (2 or more OH groups) component comprising at least about 40 wt% PO3G, based on the weight of the polyol component; (b) a polyisocyanate component comprising a diisocyanate; and (c) a hydrophilic reactant comprising a compound selected from the group consisting of (i) a mono or diisocyanate containing an ionic and/or ionizable group, and (ii) an isocyanate reactive ingredient containing an ionic and/or ionizable group. These components are reacted to form an isocyanate-functional prepolymer with ionic and/or ionizable functionality, which can then be chain extended and/or chain terminated as described in further detail below.

Polyol Component

As indicated above, the polyol component comprises at least about 40 wt% PO3G, more preferably at least about 50 wt% PO3G, still more preferably at least about 75 wt% PO3G, and even still more preferably at least about 90 wt% PO3G, based on the weight of the polyol component.

In one embodiment, the PO3G may be blended with other oligomeric and/or polymer polyfunctional isocyanate-reactive compounds such as, for example, polyols, polyamines, polythiols, polythioamines, polyhydroxythiols and polyhydroxylamines. When blended, it is preferred to use difunctional components and, more preferably, one or more diols including, for example, polyether diols, polyester diols, polycarbonate diols, polyacrylate diols, polyolefin diols and silicone diols.

In this embodiment, the PO3G is preferably blended with about 60 wt% or less, more preferably about 50 wt% or less, still more preferably about 25 wt% or less, and even still more preferably about 10 wt% or less, of the other isocyanate-reactive compounds.

Polytrimethylene Ether Glycol (PO3G)

Polytrimethylene ether glycols are oligomers and polymers in which at least 50% of the repeating units are trimethylene ether units. More preferably from about 75% to 100%, still more preferably from about 90% to 100%, and even more preferably from about 99% to 100%, of the repeating units are trimethylene ether units.

Polytrimethylene ether glycols are preferably prepared by polycondensation of monomers comprising 1,3-propanediol, thus resulting in polymers or copolymers containing $-(CH_2CH_2CH_2O)-$ linkage (e.g, trimethylene ether repeating units). As indicated above, at least 50% of the repeating units are trimethylene ether units.

In addition to the trimethylene ether units, lesser amounts of other units, such as other polyalkylene ether repeating units, may be present also. In the context of this disclosure, the term "polytrimethylene ether glycol" encompasses polytrimethylene ether glycol made from essentially pure 1,3-propanediol, as well as those oligomers and polymers (including those described below) containing up to about 50% by weight of comonomers.

The 1,3-propanediol employed for preparing the polytrimethylene ether glycols may be obtained by any of the various well known chemical routes or by biochemical transformation routes. Most preferably, the 1,3-propanediol is obtained biochemically from a renewable source ("biologically-derived" 1,3-propanediol).

The most preferred source of 1,3-propanediol is via a fermentation process using a renewable biological source. As an illustrative example of a starting material from a renewable source, biochemical routes to 1,3-propanediol (PDO) have been described that utilize feedstocks produced from biological and renewable resources such as corn feed stock. For example, bacterial strains able to convert glycerol into 1,3-propanediol are found in the species *Klebsiella, Citrobacter, Clostridium,* and *Lactobacillus.* The technique is disclosed in several patents, including US5633362, US5686276 and US5821092. For example, US5821092 discloses, *inter alia,* a process for the biological production of 1,3-propanediol from glycerol using recombinant organisms. The process incorporates E. *coli* bacteria, transformed witch a heterologous pdu diol dehydratase gene, having specificity for 1,2-propanediol. The transformed E. *coli* is grown in the presence of glycerol as a carbon source and 1,3-propanediol is isolated from the growth media. Since both bacteria and yeasts can convert glucose (e.g., corn sugar) or other carbohydrates to glycerol, the processes disclosed in these publications provide a rapid, inexpensive and environmentally responsible source of 1,3-propanediol monomer.

The biologically-derived 1,3-propanediol, such as produced by the processes described and referenced above, contains carbon from the atmospheric carbon dioxide incorporated by plants, which compose the feedstock for the production of the 1,3-propanediol. In this way, the biologically-derived 1,3-propanediol preferred for use in the context of the present invention contains only renewable carbon, and not fossil fuel-based or petroleum-based carbon. The polytrimethylene ether glycol and personal care compositions of the present invention utilizing the biologically-derived 1,3-propanediol, therefore, have less impact on the environment as the 1,3-propanediol used in the compositions does not deplete diminishing fossil fuels and, upon degradation, releases carbon back to the atmosphere for use by plants once again. Thus, the compositions present invention can be characterized as more natural and having less environmental impact than similar compositions comprising petroleum based glycols.

The biologically-derived 1,3-propanediol, and polytrimethylene ether glycols, may be distinguished from similar compounds produced from a petrochemical source or from fossil fuel carbon by dual carbon-isotopic finger printing. This method usefully distinguishes chemically-identical materials, and apportions carbon in the copolymer by source (and possibly year) of growth of the biospheric (plant) component. The isotopes, $^{14}C$ and $^{13}C$ , bring complementary information to this problem. The radiocarbon dating isotope ($^{14}C$), with its nuclear half life of 5730 years, clearly allows one to apportion specimen carbon between fossil ("dead") and biospheric ("alive") feedstocks (Currie, L. A. "Source Apportionment of Atmospheric Particles," Characterization of Environmental Particles, J. Buffle and H.P. van Leeuwen, Eds., 1 of Vol. I of the IUPAC Environmental Analytical Chemistry Series (Lewis Publishers, Inc) (1992) 3-74). The basic assumption in radiocarbon dating is that the constancy of $^{14}C$ concentration in the atmosphere leads to the constancy of $^{14}C$ in living organisms. When dealing with an isolated sample, the age of a sample can be deduced approximately by the relationship

$$t = (-5730/0.693)\ln(A/A_0)$$

where t = age, 5730 years is the half-life of radiocarbon, and A and $A_0$ are the specific $^{14}C$ activity of the sample and of the modern standard, respectively (Hsieh, Y., Soil Sci. Soc. Am J., 56, 460, (1992)). However, because of atmospheric nuclear testing since 1950 and the burning of fossil fuel since 1850, $^{14}C$ has acquired a second, geochemical time characteristic. Its concentration in atmospheric $CO_2$, and hence in the living biosphere, approximately doubled at the peak of nuclear testing, in the mid-1960s. It has since been gradually returning to the steady-state cosmogenic (atmospheric) baseline isotope rate ($^{14}C/^{12}C$) of ca. $1.2 \times 10^{-12}$, with an approximate relaxation "half-life" of 7-10 years. (This latter half-life must not be taken literally; rather, one must use the detailed atmospheric nuclear input/decay function to

trace the variation of atmospheric and biospheric $^{14}C$ since the onset of the nuclear age.) It is this latter biospheric $^{14}C$ time characteristic that holds out the promise of annual dating of recent biospheric carbon. $^{14}C$ can be measured by accelerator mass spectrometry (AMS), with results given in units of "fraction of modern carbon" ($f_M$). $f_M$ is defined by National Institute of Standards and Technology (NIST) Standard Reference Materials (SRMs) 4990B and 4990C, known as oxalic acids standards HOxI and HOxII, respectively. The fundamental definition relates to 0.95 times the $^{14}C/^{12}C$ isotope ratio HOxI (referenced to AD 1950). This is roughly equivalent to decay-corrected pre-Industrial Revolution wood. For the current living biosphere (plant material), fM ≈1.1.

The stable carbon isotope ratio ($^{13}C/^{12}C$) provides a complementary route to source discrimination and apportionment. The $^{13}C/^{12}C$ ratio in a given bio-sourced material is a consequence of the $^{13}C/^{12}C$ ratio in atmospheric carbon dioxide at the time the carbon dioxide is fixed and also reflects the precise metabolic pathway. Regional variations also occur. Petroleum, $C_3$ plants (the broad-leaf), $C_4$ plants (the grasses), and marine carbonates all show significant differences in $^{13}C/^{12}C$ and the corresponding $\delta^{13}C$ values. Furthermore, lipid matter of $C_3$ and $C_4$ plants analyze differently than materials derived from the carbohydrate components of the same plants as a consequence of the metabolic pathway. Within the precision of measurement, $^{13}C$ shows large variations due to isotopic fractionation effects, the most significant of which for the instant invention is the photosynthetic mechanism. The major cause of differences in the carbon isotope ratio in plants is closely associated with differences in the pathway of photosynthetic carbon metabolism in the plants, particularly the reaction occurring during the primary carboxylation, i.e., the initial fixation of atmospheric $CO_2$. Two large classes of vegetation are those that incorporate the "$C_3$" (or Calvin-Benson) photosynthetic cycle and those that incorporate the "$C_4$" (or Hatch-Slack) photosynthetic cycle. $C_3$ plants, such as hardwoods and conifers, are dominant in the temperate climate zones. In $C_3$ plants, the primary $CO_2$ fixation or carboxylation reaction involves the enzyme ribulose-1,5-diphosphate carboxylase and the first stable product is a 3-carbon compound. $C_4$ plants, on the other hand, include such plants as tropical grasses, corn and sugar cane. In $C_4$ plants, an additional carboxylation reaction involving another enzyme, phosphoenol-pyruvate carboxylase, is the primary carboxylation reaction. The first stable carbon compound is a 4-carbon acid, which is subsequently decarboxylated. The $CO_2$ thus released is refixed by the $C_3$ cycle.

Both $C_4$ and $C_3$ plants exhibit a range of $^{13}C/^{12}C$ isotopic ratios, but typical values are ca. -10 to -14 per mil ($C_4$) and -21 to -26 per mil ($C_3$) (Weber et al., J. Agric. Food Chem., 45, 2942 (1997)). Coal and petroleum fall generally in this latter range. The $^{13}C$ measurement scale was originally defined by a zero set by pee dee belemnite (PDB) limestone, where values are given in parts per thousand deviations from this material. The "$\delta^3C$" values are in parts per thousand (per mil), abbreviated ‰, and are calculated as follows:

$$\delta^{13}C \equiv \frac{(^{13}C/^{12}C)sample - (^{13}C/^{12}C)standard}{(^{13}C/^{12}C)standard} \times 1000‰$$

Since the PDB reference material (RM) has been exhausted, a series of alternative RMs have been developed in cooperation with the IAEA, USGS, NIST, and other selected international isotope laboratories. Notations for the per mil deviations from PDB is $\delta^{13}C$. Measurements are made on $CO_2$ by high precision stable ratio mass spectrometry (IRMS) on molecular ions of masses 44, 45 and 46.

Biologically-derived 1,3-propanediol, and compositions comprising biologically-derived 1,3-propanediol, therefore, may be completely distinguished from their petrochemical derived counterparts on the basis of $^{14}C$ ($f_M$) and dual carbon-isotopic fingerprinting, indicating new compositions of matter. The ability to distinguish these products is beneficial in tracking these materials in commerce. For example, products comprising both "new" and "old" carbon isotope profiles may be distinguished from products made only of "old" materials. Hence, the instant materials may be followed in commerce on the basis of their unique profile and for the purposes of defining competition, for determining shelf life, and especially for assessing environmental impact.

Preferably the 1,3-propanediol used as the reactant or as a component of the reactant will have a purity of greater than about 99%, and more preferably greater than about 99.9%, by weight as determined by gas chromatographic analysis. Particularly preferred are the purified 1,3-propanediols as disclosed in US20040260125A1, US20040225161A1 and US20050069997A1, and polytrimethylene ether glycol made therefrom as disclosed in US20050020805A1.

The purified 1,3-propanediol preferably has the following characteristics:

(1) an ultraviolet absorption at 220 nm of less than about 0.200, and at 250 nm of less than about 0.075, and at 275 nm of less than about 0.075; and/or

(2) a composition having L*a*b* "b*" color value of less than about 0.15 (ASTM D6290), and an absorbance at 270 nm of less than about 0.075; and/or

(3) a peroxide composition of less than about 10 ppm; and/or

(4) a concentration of total organic impurities (organic compounds other than 1,3-propanediol) of less than about 400 ppm, more preferably less than about 300 ppm, and still more preferably less than about 150 ppm, as measured by gas chromatography.

The starting material for making polytrimethylene ether glycol will depend on the desired polytrimethylene ether glycol, availability of starting materials, catalysts, equipment, etc., and comprises "1,3-propanediol reactant." By "1,3-propanediol reactant" is meant 1,3-propanediol, and oligomers and prepolymers of 1,3-propanediol preferably having a degree of polymerization of 2 to 9, and mixtures thereof. In some instances, it may be desirable to use up to 10% or more of low molecular weight oligomers where they are available. Thus, preferably the starting material comprises 1,3-propanediol and the dimer and trimer thereof. A particularly preferred starting material is comprised of about 90% by weight or more 1,3-propanediol, and more preferably 99% by weight or more 1,3-propanediol, based on the weight of the 1,3-propanediol reactant.

Polytrimethylene ether glycol can be made via a number of processes known in the art, such as disclosed in US6977291 and US6720459. A preferred process is as set forth in previously incorporated US20050020805A1.

As indicated above, polytrimethylene ether glycol may contain lesser amounts of other polyalkylene ether repeating units in addition to the trimethylene ether units. The monomers for use in preparing polytrimethylene ether glycol can, therefore, contain up to 50% by weight (preferably about 20 wt% or less, more preferably about 10 wt% or less, and still more preferably about 2 wt% or less), of comonomer diols in addition to the 1,3-propanediol reactant. Comonomer diols that are suitable for use in the process include aliphatic diols, for example, ethylene glycol, 1,6-hexanediol, 1,7-heptanediol, 1,8-octanediol, 1,9-nonanediol, 1,10-decanediol, 1,12-dodecanediol, 3,3,4,4,5,5-hexafluro-1,5-pentanediol, 2,2,3,3,4,4,5,5-octafluoro-1,6-hexanediol, and 3,3,4,4,5,5,6,6,7,7,8,8,9,9,10,10-hexadecafluoro-1,12-dodecanediol; cycloaliphatic diols, for example, 1,4-cyclohexanediol, 1,4-cyclohexanedimethanol and isosorbide; and polyhydroxy compounds, for example, glycerol, trimethylolpropane, and pentaerythritol. A preferred group of comonomer diols is selected from the group consisting of ethylene glycol, 2-methyl-1,3-propanediol, 2,2-dimethyl-1,3-propanediol; 2,2-diethyl-1,3-propanediol, 2-ethyl-2-(hydroxymethyl)-1,3-propanediol, $C_6$-$C_{10}$ diols (such as 1,6-hexanediol, 1,8-octanediol and 1,10-decanediol) and isosorbide, and mixtures thereof. A particularly preferred diol other than 1,3-propanediol is ethylene glycol, and $C_6$-$C_{10}$ diols can be particularly useful as well.

One preferred polytrimethylene ether glycol containing comonomers is poly(trimethylene-ethylene ether) glycol such as described in US2004/0030095A1. Preferred poly(trimethylene-co-ethylene ether) glycols are prepared by acid catalyzed polycondensation of from 50 to about 99 mole% (preferably from about 60 to about 98 mole%, and more preferably from about 70 to about 98 mole%) 1,3-propanediol and up to 50 to about 1 mole% (preferably from about 40 to about 2 mole%, and more preferably from about 30 to about 2 mole%) ethylene glycol.

Polytrimethylene ether glycols useful in practicing this invention can contain small amounts of other repeat units, for example, from aliphatic or aromatic diacids or diesters, such as described in US6608168. This type of polytrimethylene ether glycol can also be called a "random polytrimethylene ether ester", and can be prepared by polycondensation of 1,3-propanediol reactant and about 10 to about 0.1 mole% of aliphatic or aromatic diacid or esters thereof, such as terephthalic acid, isophthalic acid, bibenzoic acid, naphthalic acid, bis(p-carboxyphenyl)methane, 1,5-naphthalene dicarboxylic acid, 2,6-naphthalene dicarboxylic acid, 2,7-naphthalene dicarboxylic acid, 4,4'-sulfonyl dibenzoic acid, p-(hydroxyethoxy)benzoic acid, and combinations thereof, and dimethyl terephthalate, bibenzoate, isophthlate, naphthalate and phthalate; and combinations thereof. Of these, terephthalic acid, dimethyl terephthalate and dimethyl isophthalate are preferred.

The polytrimethylene ether glycols preferred for use herein generally have a number average molecular weight from about 200 to about 5000, and preferably from about 200 to about 2000. The polytrimethylene ether glycols preferred for use herein are typically polydisperse polymers having a polydispersity of preferably from about 1.0 to about 2.2, more preferably from about 1.2 to about 2.0, and still more preferably from about 1.2 to about 1.8.

The polytrimethylene ether glycols for use in the present invention preferably have a color value of less than about 100 APHA, and more preferably less than about 50 APHA.

Polytrimethylene ether glycol as described above should in general have low acute oral toxicity, and not be a skin or eye irritant, or a skin sensitizer.

<u>Other Isocyanate-Reactive Components</u>

As indicated above, the PO3G may be blended with other polyfunctional isocyanate-reactive components, preferably up to about 60 wt%, most notably oligomeric and/or polymeric polyols.

Suitable polyols contain at least two hydroxyl groups, and preferably have a molecular weight of from about 60 to about 6000. Of these, the polymeric polyols are best defined by the number average molecular weight, and can

range from about 200 to about 6000, preferably from about 300 to about 3000, and more preferably from about 500 to about 2500. The molecular weights can be determined by hydroxyl group analysis (OH number).

Examples of polymeric polyols include polyesters, polyethers, polycarbonates, polyacetals, poly(meth)acrylates, polyester amides, polythioethers, and mixed polymers such as a polyester-polycarbonates where both ester and carbonate linkages are found in the same polymer. Also included are vegetable-based polyols. A combination of these polymers can also be used. For examples, a polyester polyol and a poly(meth)acrylate polyol may be used in the same polyurethane synthesis.

Suitable polyester polyols include reaction products of polyhydric, preferably dihydric alcohols to which trihydric alcohols may optionally be added, and polybasic (preferably dibasic) carboxylic acids. Instead of these polycarboxylic acids, the corresponding carboxylic acid anhydrides or polycarboxylic acid esters of lower alcohols or mixtures thereof may be used for preparing the polyesters.

The polycarboxylic acids may be aliphatic, cycloaliphatic, aromatic and/or heterocyclic or mixtures thereof and they may be substituted, for example, by halogen atoms, and/or unsaturated. The following are mentioned as examples: succinic acid; adipic acid; suberic acid; azelaic acid; sebacic acid; 1,12-dodecyldioic acid; phthalic acid; isophthalic acid; trimellitic acid; phthalic acid anhydride; tetrahydrophthalic acid anhydride; hexahydrophthalic acid anhydride; tetrachlorophthalic acid anhydride; endomethylene tetrahydrophthalic acid anhydride; glutaric acid anhydride; maleic acid; maleic acid anhydride; fumaric acid; dimeric and trimeric fatty acids such as oleic acid, which may be mixed with monomeric fatty acids; dimethyl terephthalates and bis-glycol terephthalate.

Suitable polyhydric alcohols include, e.g., ethylene glycol; propylene glycol-(1,2) and -(1,3); butylene glycol-(1,4) and -(1,3); hexanediol-(1,6); octanediol-(1,8); neopentyl glycol; cyclohexanedimethanol (1,4-bis-hydroxymethylcyclohexane); 2-methyl-1,3-propanediol; 2,2,4-trimethyl-1, 3-pentanediol; diethylene glycol, triethylene glycol; tetraethylene glycol; polyethylene glycol; dipropylene glycol; polypropylene glycol; dibutylene glycol and polybutylene glycol; glycerine; trimethylolpropane; ether glycols thereof; and mixtures thereof. The polyester polyols may also contain a portion of carboxyl end groups. Polyesters of lactones, for example, epsilon-caprolactone, or hydroxycarboxylic acids, for example, omega-hydroxycaproic acid, may also be used.

Preferable polyester diols for blending with PO3G are hydroxyl-terminated poly(butylene adipate), poly(butylene succinate), poly(ethylene adipate), poly(1,2-proylene adipate), poly(trimethylene adipate), poly(trimethylene succinate), polylactic acid ester diol and polycaprolactone diol. Other hydroxyl terminated polyester diols are copolyethers comprising repeat units derived from a diol and a sulfonated dicarboxylic acid and prepared as described in US6316586. The preferred sulfonated dicarboxylic acid is 5-sulfo-isophthalic acid, and the preferred diol is 1,3-propanediol.

Suitable polyether polyols are obtained in a known manner by the reaction of starting compounds that contain reactive hydrogen atoms with alkylene oxides such as ethylene oxide, propylene oxide, butylene oxide, tetrahydrofuran, styrene oxide, epichlorohydrin or mixtures of these. Suitable starting compounds containing reactive hydrogen atoms include the polyhydric alcohols set forth above and, in addition, water, methanol, ethanol, 1,2,6-hexane triol, 1,2,4-butane triol, trimethylol ethane, pentaerythritol, mannitol, sorbitol, methyl glycoside, sucrose, phenol, isononyl phenol, resorcinol, hydroquinone, 1,1,1- and 1,1,2-tris-(hydroxylphenyl)-ethane, dimethylolpropionic acid or dimethylolbutanoic acid.

Polyethers that have been obtained by the reaction of starting compounds containing amine compounds can also be used. Examples of these polyethers as well as suitable polyhydroxy polyacetals, polyhydroxy polyacrylates, polyhydroxy polyester amides, polyhydroxy polyamides and polyhydroxy polythioethers, are disclosed in US4701480.

Preferred polyether diols for blending with PO3G are polyethylene glycol, poly(1,2-propylene glycol), polytetramethylene glycol, copolyethers such as tetrahydrofuran/ethylene oxide and tetrahydrofuran/propylene oxide copolymers, and mixtures thereof.

Polycarbonates containing hydroxyl groups include those known, per se, such as the products obtained from the reaction of diols such as propanediol-(1,3), butanediol-(1,4) and/or hexanediol-(1,6), diethylene glycol, triethylene glycol or tetraethylene glycol, higher polyether diols with phosgene, diarylcarbonates such as diphenylcarbonate, dialkylcarbonates such as diethylcarbonate or with cyclic carbonates such as ethylene or propylene carbonate. Also suitable are polyester carbonates obtained from the above-mentioned polyesters or polylactones with phosgene, diaryl carbonates, dialkyl carbonates or cyclic carbonates.

Polycarbonate diols for blending are preferably selected from the group consisting of polyethylene carbonate diol, polytrimethylene carbonate diol, polybutylene carbonate diol and polyhexylene carbonate diol.

Poly(meth)acrylates containing hydroxyl groups include those common in the art of addition polymerization such as cationic, anionic and radical polymerization and the like. Examples are alpha-omega diols. An example of these type of diols are those which are prepared by a "living" or "control" or chain transfer polymerization processes which enables the placement of one hydroxyl group at or near the termini of the polymer. US6248839 and US5990245 have examples of protocol for making terminal diols. Other di-NCO reactive poly(meth)acrylate terminal polymers can be used. An example would be end groups other than hydroxyl such as amino or thiol, and may also include mixed end groups with hydroxyl.

Polyolefin diols are available from Shell as KRATON LIQUID L and Mitsubishi Chemical as POLYTAIL H.

Silicone glycols are well known, and representative examples are described in US4647643.

In some instances, vegetable oils may be the preferred blending component because of their biological origin and biodegradability. Examples of vegetable oils include but are not limited to sunflower oil, canola oil, rapeseed oil, corn oil, olive oil, soybean oil, castor oil and mixtures thereof. These oils are either partial or fully hydrogenated. Commercially available examples of such vegetable oils include Soyol R2-052-G (Urethane Soy Systems) and Pripol 2033 (Uniqema).

Other optional compounds for preparing the NCO-functional prepolymer include lower molecular weight, at least difunctional NCO-reactive compounds having an average molecular weight of up to about 400. Examples include the dihydric and higher functional alcohols, which have previously been described for the preparation of the polyester polyols and polyether polyols.

In addition to the above-mentioned components, which are preferably difunctional in the isocyanate polyaddition reaction, mono-functional and even small portions of trifunctional and higher functional components generally known in polyurethane chemistry, such as trimethylolpropane or 4-isocyanantomethyl-1,8-octamethylene diisocyanate, may be used in cases in which branching of the NCO prepolymer or polyurethane is desired.

It is, however, preferred that the NCO-functional prepolymers should be substantially linear, and this may be achieved by maintaining the average functionality of the prepolymer starting components at or below 2:1.

Similar NCO reactive materials can be used as described for hydroxy containing compounds and polymers, but which contain other NCO reactive groups. Examples would be dithiols, diamines, thioamines, and even hydroxythiols and hydroxylamines. These can either be compounds or polymers with the molecular weights or number average molecular weights as described for the polyols.

Other optional compounds include isocyanate-reactive compounds containing self-condensing moieties. The content of these compounds are dependent upon the desired level of self-condensation necessary to provide the desirable resin properties. 3-amino-1-triethoxysilyl-propane is an example of a compound that will react with isocyanates through the amino group and yet self-condense through the silyl group when inverted into water.

Other optional compounds include isocyanate-reactive compounds containing non-condensable silanes and/or fluorocarbons with isocyanate reactive groups, which can be used in place of or in conjunction with the isocyanate-reactive compounds. US5760123 and US6046295 list examples of methods for use of these optional silahe/fluoro-containing compounds.

Polyisocyanate Component

Suitable polyisocyanates are those that contain aromatic, cycloaliphatic and/or aliphatic groups bound to the isocyanate groups. Mixtures of these compounds may also be used. Preferred are compounds with isocyanates bound to a cycloaliphatic or aliphatic moieties. If aromatic isocyanates are used, cycloaliphatic or aliphatic isocyanates are preferably present as well.

Diisocyanates are preferred, and any diisocyanate useful in preparing polyurethanes and/or polyurethane-ureas from polyether glycols, diols and/or amines can be used in this invention.

Examples of suitable diisocyanates include, but are not limited to, 2,4-toluene diisocyanate (TDI); 2,6-toluene diisocyanate; trimethyl hexamethylene diisocyanate (TMDI); 4,4'-diphenylmethane diisocyanate (MDI); 4,4'-dicyclohexylmethane diisocyanate ($H_{12}$MDI); 3,3'-dimethyl-4,4'-biphenyl diisocyanate (TODI); Dodecane diisocyanate ($C_{12}$DI); m-tetramethylene xylylene diisocyanate (TMXDI); 1,4-benzene diisocyanate; trans-cyclohexane-1,4-diisocyanate; 1,5-naphthalene diisocyanate (NDI); 1,6-hexamethylene diisocyanate (HDI); 4,6-xylyene diisocyanate; isophorone diisocyanate (IPDI); and combinations thereof. IPDI and TMXDI are preferred.

Small amounts, preferably less than about 10 wt% based on the weight of the diisocyanate, of monoisocyanates or polyisocyanates can be used in mixture with the diisocyanate. Examples of useful monoisocyanates include alkyl isocyanates such as octadecyl isocyanate and aryl isocyanates such as phenyl isocyanate. An example of a polyisocyanate is triisocyanatotoluene HDI trimer (Desmodur 3300), and polymeric MDI (Mondur MR and MRS).

Ionic Reactants

The hydrophilic reactant contains ionic and/or ionizable groups (potentially ionic groups). Preferably, these reactants will contain one or two, more preferably two, isocyanate reactive groups, as well as at least one ionic or ionizable group.

Examples of ionic dispersing groups include carboxylate groups (-COOM), phosphate groups ($-OPO_3\ M_2$), phosphonate groups ($-PO_3\ M_2$), sulfonate groups ($-SO_3$ M), quaternary ammonium groups ($-NR_3$ Y, wherein Y is a monovalent anion such as chlorine or hydroxyl), or any other effective ionic group. M is a cation such as a monovalent metal ion (e.g., $Na^+$, $K^+$, $Li^+$, etc.), $H^+$, $NR_4^+$, and each R can be independently an alkyl, aralkyl, aryl, or hydrogen. These ionic dispersing groups are typically located pendant from the polyurethane backbone.

The ionizable groups in general correspond to the ionic groups, except they are in the acid (such as carboxyl -COOH) or base (such as primary, secondary or tertiary amine $-NH_2$, -NRH, or $-NR_2$) form. The ionizable groups are such that they are readily converted to their ionic form during the dispersion/polymer preparation process as discussed below.

The ionic or potentially ionic groups are chemically incorporated into the polyurethane in an amount to provide an ionic group content (with neutralization as needed) sufficient to render the polyurethane dispersible in the aqueous medium of the dispersion. Typical ionic group content will range from about 5 up to about 210 milliequivalents (meq), preferably from about 10 to about 140 meq, more preferably from about 20 to about 120 meq, and still more preferably from about 30 to about 90 meq, per 100 g of polyurethane.

Suitable compounds for incorporating these groups include (1) monoisocyanates or diisocyanates which contain ionic and/or ionizable groups, and (2) compounds which contain both isocyanate reactive groups and ionic and/or ionizable groups. In the context of this disclosure, the term "isocyanate reactive groups" is taken to include groups well known to those of ordinary skill in the relevant art to react with isocyanates, and preferably hydroxyl, primary amino and secondary amino groups.

Examples of isocyanates that contain ionic or potentially ionic groups are sulfonated toluene diisocyanate and sulfonated diphenylmethanediisocyanate.

With respect to compounds which contain isocyanate reactive groups and ionic or potentially ionic groups, the isocyanate reactive groups are typically amino and hydroxyl groups. The potentially ionic groups or their corresponding ionic groups may be cationic or anionic, although the anionic groups are preferred. Preferred examples of anionic groups include carboxylate and sulfonate groups. Preferred examples of cationic groups include quaternary ammonium groups and sulfonium groups.

The neutralizing agents for converting the ionizable groups to ionic groups are described in the preceding incorporated publications, and are also discussed hereinafter. Within the context of this invention, the term "neutralizing agents" is meant to embrace all types of agents that are useful for converting ionizable groups to the more hydrophilic ionic (salt) groups.

Suitable compounds for incorporating the previously discussed carboxylate, sulfonate and quaternary nitrogen groups are described in US3479310, US4303774, US4108814 and US4408008.

Suitable compounds for incorporating tertiary sulfonium groups are described in US3419533.

Sulfonate groups for incorporation into the polyurethanes preferably are the diol sulfonates as disclosed in previously incorporated US4108814. Suitable diol sulfonate compounds also include hydroxyl terminated copolyethers comprising repeat units derived from a diol and a sulfonated dicarboxylic acid and prepared as described in previously incorporated US6316586. The preferred sulfonated dicarboxylic acid is 5-sulfo-isophthalic acid, and the preferred diol is 1,3-propanediol. Suitable sulfonates also include $H_2N-CH_2-CH_2-NH-(CH_2)_r-SO_3Na$, where r=2 or 3; and $HO-CH_2-CH_2-C(SO_3Na)-CH_2-OH$.

Examples of carboxylic group-containing compounds are the hydroxy-carboxylic acids corresponding to the formula $(HO)_xQ(COOH)_y$ wherein Q represents a straight or branched, hydrocarbon radical containing 1 to 12 carbon atoms, x is 1 or 2 (preferably 2), and y is 1 to 3 (preferably 1 or 2).

Examples of these hydroxy-carboxylic acids include citric acid, tartaric acid and hydroxypivalic acid.

The preferred acids are those of the above-mentioned formula wherein x=2 and y=1. These dihydroxy alkanoic acids are described in US3412054. The preferred group of dihydroxy alkanoic acids are the σ,σ-dimethylol alkanoic acids represented by the structural formula $R^2$-C-($CH_2$OHh-COOH, wherein $R^2$ is hydrogen or an alkyl group containing 1 to 8 carbon atoms. Examples of these ionizable diols include but are not limited to dimethylolacetic acid, 2,2'-dimethylolbutanoic acid, 2,2'-dimethylolpropionic acid, and 2,2'-dimethylolbutyric acid. The most preferred dihydroxy alkanoic acids is 2,2'-dimethylolpropionic acid ("DMPA").

When the ionic stabilizing groups are acids, the acid groups are incorporated in an amount sufficient to provide an acid group content, known by those skilled in the art as acid number (mg KOH per gram solid polymer), of at least about 5, preferably at least about 10 milligrams KOH per 1.0 gram of polyurethane. The upper limit for the acid number is about 90, and preferably about 60.

Suitable carboxylates also include $H_2N-(CH_2)_4-CH(CO_2H)-NH_2$, and $H_2N-CH_2-CH_2-NH-CH_2-CH_2-CO_2Na$.

In addition to the foregoing, cationic centers such as tertiary amines with one alkyl and two alkylol groups may also be used as the ionic or ionizable group.

## Polyurethane and Dispersion Preparation

The process of preparing the dispersions used in the invention begins with preparation of the polyurethane, which can be prepared by mixture or stepwise methods.

In the mixture process, an isocyanate-terminated polyurethane prepolymer is prepared by mixing the polyol component, the ionic reactants and solvent, and then adding polyisocyanate component to the mixture. This reaction is

conducted at from about 40°C to about 100°C, and more preferably from about 50°C to about 90°C. The preferred ratio of isocyanate to isocyanate reactive groups is from about 1.3:1 to about 1.05:1, and more preferably from about 1.25:1 to about 1.1:1. When the targeted percent isocyanate is reached (typically an isocyanate content of about 1 to about 20%, preferably about 1 to about 10% by weight, based on the weight of prepolymer solids), then the optional chain terminator can be added, as well as a base or acid to neutralize ionizable groups incorporated from the ionic reactant.

If some cases, addition of neutralization agent, preferably tertiary amines, may be beneficial during early stages of the polyurethane synthesis. Alternately, advantages may be achieved via the addition of the neutralization agent, preferably alkali base, simultaneously along with the water of inversion at high shear.

In the stepwise method, an isocyanate-terminated polyurethane prepolymer is prepared by dissolving the ionic reactant in solvent, and then adding the polyisocyanate component to the mixture. Once the initial percent isocyanate target is reached, the polyol component is added. This reaction is conducted at from about 40°C to about 100°C, and more preferably from about 50°C to about 90°C. The preferred ratio of isocyanate to isocyanate reactive groups is from about 1.3:1 to about 1.05:1, and more preferably from about 1.25:1 to about 1.1:1. Alternately, the polyol component may be reacted in the first step, and the ionic reactant may be added after the initial percent isocyanate target is reached. When the final targeted percent isocyanate is reached (typically an isocyanate content of about 1 to about 20%, preferably about 1 to about 10% by weight, based on the weight of prepolymer solids), then the optional chain terminator may be added, as well as a base or acid to neutralize ionizable groups incorporated from the ionic reactant.

The resulting polyurethane solution is then converted to an aqueous polyurethane dispersion via the addition of water under shear, as discussed in further detail below. The optional chain extender is added at this point, if the chain terminator is omitted or reduced to leave sufficient isocyanate functionality. Chain extension is typically performed at 30°C to 60°C under aqueous conditions. If present, the volatile solvent is distilled under reduced pressure.

Catalysts are often necessary to prepare the polyurethanes, and may provide advantages in their manufacture. The catalysts most widely used are tertiary amines such as tertiary ethylamine, organo-tin compounds such as stannous octoate, dibutyltin dioctoate, dibutyltin dilaurate, organo-titanates such as TYZOR® TPT or TYZOR® TBT (E.I. du Pont de Nemours and Company), organo-zirconates, and mixtures thereof.

Preparation of the polyurethane for subsequent conversion to a dispersion is facilitated by using solvent. Suitable solvents are those that are miscible with water and inert to isocyanates and other reactants utilized in forming the polyurethanes. If it is desired to prepare a solvent-free dispersion, then it is preferable to use a solvent with a high enough volatility to allow removal by distillation. Typical solvents useful in the practice of the invention are acetone, methyl ethyl ketone, toluene, and N-methyl pyrollidone. Preferably the amount of solvent used in the reaction will be from about 10% to about 50%, more preferably from about 20% to about 40% of the weight.

Polymerizable vinyl compounds may also be used as solvents, followed by free radical polymerization after inversion, thus forming a polyurethane/acrylic hybrid dispersion, as disclosed in previously incorporated US5173526, US4644030, US5488383 and US5569705.

Optional Chain Extenders/Terminators

The polyurethanes are typical prepared by chain extending the NCO-containing prepolymers. The function of a chain extender is to increase the molecular weight of the polyurethanes. Chain extension can take place prior to addition of water in the process, but typically takes place by combining the NCO-containing prepolymer, chain extender, water and other optional components under agitation.

The reactants used to prepare the polyurethanes may contain a chain extender, which is typically a polyol, polyamine or aminoalcohol. When polyol chain extenders are used, urethane linkages form as the hydroxyl groups of the polyol react with isocyanates. When polyamine chain extenders are used, urea linkages are formed as the amine groups react with the isocyanates. Both structural types are included within the meaning of "polyurethanes".

Preferably, the optional chain extender will be polyamine. Suitable polyamines for preparing the at least partially blocked polyamines have an average functionality, i.e., the number of amine nitrogens per molecule, of 2 to 6, preferably 2 to 4 and more preferably 2 to 3. The desired functionalities can be obtained by using mixtures of polyamines containing primary or secondary amino groups. The polyamines are generally aromatic, aliphatic or alicyclic amines and contain between 1 to 30, preferably 2 to 15 and more preferably 2 to 10 carbon atoms. These polyamines may contain additional substituents provided that they are not as reactive with isocyanate groups as the primary or secondary amines. These same polyamines can be partially or wholly blocked polyamines.

Diamine chain extenders useful in making the polyurethanes used in the invention include 1,2-ethylenediamine; 1,6-hexanediamine; 1,2-propanediamine; 4,4'-methylene-bis(3-chloroaniline) (also known as 3,3'-dichloro-4,4'-diaminodiphenylmethane) (MOCA or Mboca); isophorone diamine; dimethylthiotoluenediamine (DMTDA); 4,4'-diaminodiphenylmethane (DDM); 1,3-diaminobenzene; 1,4-diaminobenzene; 3,3'-dimethoxy-4,4'-diamino biphenyl; 3,3'-dimethyl-4,4'-diamino biphenyl; 4,4'-diamino biphenyl; 3,3'-dichloro-4,4'-diamino biphenyl; hydrazine; and combinations thereof. Polyamines such as diethylene triamine (DETA), triethylene tetraamine (TETA), tetraethylene pentamine and pentaethylene hexamine are also useful.

Suitable polyamine chain extenders can optionally be partially or wholly blocked as disclosed in US4269748 and US4829122. These publications disclose the preparation of aqueous polyurethane dispersions by mixing NCO-containing prepolymers with at least partially blocked, diamine or hydrazine chain extenders in the absence of water and then adding the mixture to water. Upon contact with water the blocking agent is released and the resulting unblocked polyamine reacts with the NCO containing prepolymer to form the polyurethane.

Suitable blocked amines and hydrazines include the reaction products of polyamines with ketones and aldehydes to form ketimines and aldimines, and the reaction of hydrazine with ketones and aldehydes to form ketazines, aldazines, ketone hydrazones and aldehyde hydrazones. The at least partially blocked polyamines contain at most one primary or secondary amino group and at least one blocked primary or secondary amino group which releases a free primary or secondary amino group in the presence of water.

Water may also be employed as a chain extender. In this case, water will be present in a gross excess relative to the free isocyanate groups, and these ratios are not applicable since water functions as both dispersing medium and chain extender.

The reactants used to prepare the polyurethanes of the aqueous dispersions of the invention may also contain a chain terminator. The optional chain terminators control the molecular weight of the polyurethanes, and can be added before, during or after inversion of the pre-polymer.

Suitable chain terminators include amines or alcohols having an average functionality per molecule of 1, i.e., the number of primary or secondary amine nitrogens or alcohol oxygens would average 1 per molecule. The desired functionalities can be obtained by using primary or secondary amino groups. The amines or alcohols are generally aromatic, aliphatic or alicyclic and contain between 1 to 30, preferably 2 to 15 and more preferably 2 to 10 carbon atoms.

Preferred monoalcohols for use as chain terminators include $C_1$-$C_{18}$ alkyl alcohols such as n-butanol, n-octanol, and n-decanol, n-dodecanol, stearyl alcohol and $C_2$-$C_{12}$ fluorinated alcohols, and more preferably $C_1$-$C_6$ alkyl alcohols such as n-propanol, ethanol, and methanol.

Any primary or secondary monoamines reactive with isocyanates may be used as chain terminators. Aliphatic primary or secondary monoamines are preferred. Example of monoamines useful as chain terminators include but are not restricted to butylamine, hexylamine, 2-ethylhexyl amine, dodecyl amine, diiso-propanol amine, stearyl amine, dibutyl amine, dinonyl amine, bis(2-ethylhexyl) amine, diethylamine, bis(methoxyethyl)amine, N- methylstearyl amine and N-methyl aniline. A more preferred isocyanate reactive chain terminator is bis(methoxyethyl)amine.

Urethane end groups are formed when alcohol chain terminators are used; urea end groups are formed when amine chain terminators are used. Both structural types are referred to herein as "polyurethanes".

Chain terminators and chain extenders can be used together, either as mixtures or as sequential additions to the NCO-prepolymer.

The amount of chain extender/terminator employed should be approximately equivalent to the free isocyanate groups in the prepolymer, the ratio of active hydrogens in the chain extender to isocyanate groups in the prepolymer preferably being in the range from about 0.6:1 to about 1.3:1, more preferably from about 0.6:1 to about 1.1:1, and still more preferably from about 0.7:1 to about 1.1:1, and even more preferably from about 0.9:1 to about 1.1:1, on an equivalent basis. Any isocyanate groups that are not chain extended/terminated with an amine or alcohol will react with water which, as indicated above, functions as a chain extender.

<u>Neutralization</u>

When the potential cationic or anionic groups of the polyurethane are neutralized, they provide hydrophilicity to the polymer and better enable it to be stably dispersed in water. The neutralization steps may be conducted (1) prior to polyurethane formation by treating the component containing the potentially ionic group(s), or (2) after polyurethane formation, but prior to dispersing the polyurethane, or (3) concurrently with the dispersion preparation. The reaction between the neutralizing agent and the potentially ionic groups may be conducted between about 20°C and about 150°C, but is normally conducted at temperatures below about 100°C, preferably between about 30°C and about 80°C, and more preferably between about 50°C and about 70°C, with agitation of the reaction mixture.

In order to have a stable dispersion, a sufficient amount of the ionic groups (e.g., neutralized ionizable groups) must be present so that the resulting polyurethane will remain stably dispersed in the aqueous medium. Generally, at least about 70%, preferably at least about 80%, of the acid groups are neutralized to the corresponding carboxylate salt groups. Alternatively, cationic groups in the polyurethane can be quaternary ammonium groups ($-NR_3Y$, wherein Y is a monovalent anion such as chlorine or hydroxyl).

Suitable neutralizing agents for converting the acid groups to salt groups include tertiary amines, alkali metal cations and ammonia. Examples of these neutralizing agents are disclosed in previously incorporated US4701480, as well as US4501852. Preferred neutralizing agents are the trialkyl-substituted tertiary amines, such as triethyl amine, tripropyl amine, dimethylcyclohexyl amine, and dimethylethyl amine and alkali metal cations such as sodium or potassium.

Substituted amines are also useful neutralizing groups such as diethyl ethanol amine or diethanol methyl amine.
Neutralization may take place at any point in the process. Typical procedures include at least some neutralization of the prepolymer, which is then chain extended/terminated in water in the presence of additional neutralizing agent.
The final product is a stable aqueous dispersion of polyurethane particles having a solids content of up to about 60% by weight, preferably from about 15 to about 60% by weight, and more preferably from about 30 to about 40% by weight. However, it is always possible to dilute the dispersions to any minimum solids content desired.

Dispersion Preparation

In accordance with the present invention the term "aqueous polyurethane dispersion" refers to aqueous dispersions of polymers containing urethane groups, as that term is understood by those of ordinary skill in the art. These polymers also preferably incorporate hydrophilic functionality to the extent required to maintain a stable dispersion of the polymer in water (the aqueous vehicle). The compositions of the invention that are based on aqueous dispersions comprise a continuous phase comprising water, and a dispersed phase comprising polyurethane.
Following formation of the desired polyurethane, preferably in the presence of solvent as discussed above, the pH may be adjusted, if necessary, to insure conversion of ionizable groups to ionic groups (neutralization). For example, if the preferred dimethylolpropionic acid is the ionic or ionizable ingredient used in making the polyurethane, then sufficient aqueous base is added to convert the carboxyl groups to carboxylate anions.
Conversion to the aqueous dispersion is completed by addition of water. If desired, solvent can then be removed partially or substantially completely by distillation under reduced pressure. The total solids level of the aqueous dispersions are preferably in the range of from about 5 wt% to about 70 wt%, and more preferably from about 20 wt% to about 40 wt%, based on the total weight of the dispersion.
If desired, surfactant may be added to the dispersion to improve stability. The surfactant may be anionic, cationic or nonionic. If used, the preferred amount of surfactant is from about 0.1 wt% to about 2 wt%. Examples of preferred surfactants are dodecylbenzenesulfonate or TRITON X (Dow Chemical Co., Midland, MI).
The final product is a stable, aqueous polyurethane dispersion having a solids content of up to about 70% by weight, preferably from about 10% to about 60% by weight, and more preferably from about 20% to about 45% by weight. However, it is always possible to dilute the dispersions to any minimum solids content desired. The solids content of the resulting dispersion may be determined by drying the sample in an oven at 150°C for 2 hours and comparing the weights before and after drying. The particle size is generally below about 1.0 micron, and preferably between about 0.01 to about 0.5 micron. The average particle size should be less than about 0.5 micron, and preferably between about 0.01 to about 0.3 micron. The small particle size enhances the stability of the dispersed particles

Crosslinking

It is within the scope of the present invention to have some crosslinking in the polyurethane.
The means to achieve the crosslinking of the polyurethane generally relies on at least one component of the polyurethane (starting material and/or intermediate) having 3 or more functional reaction sites. Reaction of each of the 3 (or more) reaction sites will produce a crosslinked polyurethane (3-dimensional matrix). When only two reactive sites are available on each reactive components, only linear (albeit possibly high molecular weight) polyurethanes can be produced. Examples of crosslinking techniques include but are not limited to the following:

the isocyanate-reactive moiety has at least 3 reactive groups, for example polyfunctional amines or polyol;

the isocyanate has at least 3 isocyanate groups;

the prepolymer chain has at least 3 reactive sites that can react via reactions other than the isocyanate reaction, for example with amino trialkoxysilanes;

addition of a reactive component with at least 3 reactive sites to the polyurethane prior to its use, for example trifunctional epoxy crosslinkers;

addition of a water-dispersible crosslinker with oxazoline functionality;

synthesis of a polyurethane with carbonyl functionality, followed by addition of a dihydrazide compound;

and any combination of the these crosslinking methods and other crosslinking means known to those of ordinary skill in the relevant art.

Also, it is understood that these crosslinking components may only be a (small) fraction of the total reactive functionality added to the polyurethane. For example, when polyfunctional amines are added, mono- and difunctional amines may also be present for reaction with the isocyanates. The polyfunctional amine may be a minor portion of the amines.

The emulsion/dispersion stability of the crosslinked polyurethane can if needed be improved by added dispersants or emulsifiers.

When crosslinking is desired, the lower limit of crosslinking in the polyurethane is about 1 % or greater, preferably about 4% or greater, and more preferably about 10% or greater, as measured by the THF insolubles test.

The amount of crosslinking can be measured by a standard tetrahydrofuran insolubles test. For the purposes of definition herein, the tetrahydrofuran (THF) insolubles of the polyurethane dispersoid is measured by mixing 1 gram of the polyurethane dispersoid with 30 grams of THF in a pre-weighed centrifuge tube. After the solution is centrifuged for 2 hours at 17000 rpm, the top liquid layer is poured out and the non-dissolved gel in the bottom is left. The centrifuge tube with the non-dissolved gel is re-weighed after the tube is put in the oven and dried for 2 hours at 110°C.

% THF insolubles of polyurethane = (weight of tube and non-dissolved gel - weight of tube)/(sample weight * polyurethane solid %)

An alternative way to achieve an effective amount of crosslinking in the polyurethane is to choose a polyurethane that has crosslinkable sites, then crosslink those sites via self-crosslinking and/or added crosslinking agents. Examples of self-crosslinking functionality includes, for example, silyl functionality (self-condensing) available from certain starting materials as indicated above, as well as combinations of reactive functionalities incorporated into the polyurethanes, such as epoxy/hydroxyl, epoxy/acid and isocyanate/hydroxyl. Examples, of polyurethanes and complementary crosslinking agents include: (1) a polyurethane with isocyanate reactive sites (such as hydroxyl and/or amine groups) and an isocyanate crosslinking reactant, and 2) a polyurethane with unreacted isocyanate groups and an isocyanate-reactive crosslinking reactant (containing, for example, hydroxyl and/or amine groups). The complementary reactant can be added to the polyurethane, such that crosslinking can be done prior to its incorporation into a formulation.

Further details on crosslinked polyurethanes can be found, for example, in US20050215663A1.

<u>Film-Forming Polyurethanes</u>

For some applications in accordance with the present invention, such as lipsticks/lip gloss, nail polish and hair styling compositions, it is preferred that the polyurethane is film-forming. By "film-forming" is it meant that the polyurethane, after application to a surface, forms a coherent film (continuous phase) containing the active cosmetic ingredient (discontinuous phase) under application conditions.

The film-forming capabilities of a particularly polyurethane under specified applications conditions can be readily determined by a person of ordinary skill in the art without undue experimentation.

<u>Active Cosmetic Ingredients</u>

The cosmetic compositions of the invention comprise, in addition to the aqueous vehicle and polyurethane ionomer, one or more active cosmetics ingredients that provide some cosmetic benefit to the user's body, e.g., to the hair, nails and/or skin. Such materials are in general well-known to those persons of ordinary skill in the relevant cosmetic art and may include, for example, moisturizing agents, antiperspirants, anti-bacterials, sunscreen agents, insect repellents, cleaning agents, hair conditioning agents, hair styling agents, anti-dandruff agents, hair growth promoters, dyes and pigments, anesthetics, lubricants, soaps and perfumes. The cosmetic compositions in accordance with the presentinvention are intended for surface (external) application.

A wide variety of cosmetic agents are useful in the cosmetic compositions of the present invention to the extent that those agents do not interfere with the dispersibility of the polyurethane in the aqueous continuous phase.

The active cosmetic ingredients (and other ingredients of the personal care compositions as described below) can be categorized (including in multiple categories) by the benefit they provide or by their postulated mode of action. However, it is to be understood that the active cosmetic ingredients (and other ingredients) useful herein can in some instances provide more than one benefit or operate via more than one mode of action. Therefore, classifications herein are made for the sake of convenience and are not intended to limit the active to that particular application or applications listed.

Examples of substances that may suitably be included in the cosmetic compositions according to the present invention as active cosmetic ingredients include the following:

(1) perfumes and fragrances, which give rise to an olfactory response in the form of a fragrance, and deodorant perfumes which, in addition to providing a fragrance response, can also reduce body malodor;

(2) skin coolants, such as menthol, menthyl acetate, menthyl pyrrolidone carboxylate, N-ethyl-p-menthane-3-carboxamide and other derivatives of menthol, which give rise to a tactile response in the form of a cooling sensation on the skin;

(3) emollients, such as isopropylmyristate, silicone oils, mineral oils and vegetable oils, which give rise to a tactile response in the form of an increase in skin lubricity;

(4) deodorants other than perfumes, whose function is to reduce the level of or eliminate micro flora at the skin surface, especially those responsible for the development of body malodor, including precursors of deodorants;

(5) antiperspirant actives, whose function is to reduce or eliminate the appearance of perspiration at the skin surface;

(6) moisturizing agents, that keep the skin moist by either adding moisture or preventing from evaporating from the skin;

(7) sunscreen active ingredients that protect the skin and hair from UV and other harmful light rays from the sun;

(8) hair treatment agents that condition hair, clean hair, detangle hair, act as styling agents, anti-dandruff agents, hair growth promoters, hair dyes and pigments, hair perfumes, hair relaxers, hair bleaching agents, hair moisturizers, hair oil treatment agents and antifrizzing agents;

(9) beauty aids, such as powders, pigments and colorants; and

(10) medicinal agents.

Further examples of skin benefit agents include abrasives; absorbents; aesthetic components such as opacifying agents and pearlescent aids such as ethylene glycol distearate and $TiO_2$ coated mica; essential oils; skin sensates; cosmetic and drug astringents such as clove oil, menthol, camphor, eucalyptus oil, eugenol, menthyl lactate and witch hazel distillate; anti-acne agents such as resorcinol, sulfur, salicylic acid, benzoyl peroxide, erythromycin and zinc; anti-caking agents; antimicrobial agents such as iodopropyl butylcarbamate; antioxidants; cosmetic biocides; external analgesics; pH modifiers such as citric acid, sodium citrate, succinic acid, phosphoric acid, sodium hydroxide and sodium carbonate; skin bleaching and lightening agents such as hydroquinone, kojic acid, ascorbic acid, magnesium ascorbyl phosphate and ascorbyl glucosamine; skin soothing and/or healing agents such as panthenol and derivatives like ethyl panthenol, aloe vera, pantothenic acid and its derivatives, allantoin; bisabolol and dipotassium glycyrrhizinate; retinoids such as retinol palmitate); tocopheryl nicotinate; skin treating agents; vitamins and derivatives thereof; and other similar materials.

Humectants have been described as agents that control the moisture exchange between the product and air, both in the container and on the skin. Humectants have also been described as compounds that prevent drying of skin or that increase the water content of the top layers of skin (e.g., hygroscopic compounds). One potentially preferred humectant is PO3G (in and of itself) as described above.

Suitable moisturizing agents include hydrophobic agents, hydrophilic agents and combinations thereof. Examples of moisturizing agents are allantoin, glycerol, polyglycerylmethacrylate, panthenol, polyols, ceramide, borage oil (linoleic acid), tocopherol (Vitamin E), tocopherol linoleate, dimethicone, hyaluronic acid, sodium peroxylinecarbolic acid (sodium PCA), wheat protein (e.g., laurdimonium hydroxypropyl hydrolyzed wheat protein), hair keratin amino acids, panthenol; primrose oil; GLA 3 and other fish oils that may include, for example, the omega-3 and omega- 6 oils and/or linoleic acid; and flax seed oil, and mixtures thereof. Other moisturizing agents can also be used.

Numerous sunscreen agents are suitable for use in the personal care compositions of the present invention. Examples include, p-aminobenzoic acid, its salts and its derivatives, anthranilates, salicylates, cinnamic acid derivatives, dihydroxy cinnamic acid derivatives, trihydroxy cinnamic acid derivatives, dibenzalacetone, dibenzalacetophenone, naphtholsulfonates, dihydroxynaphtholic acid and its salts, coumarin derivatives, diazoles, quinine salts, quinoline derivatives, hydroxy- and methoxy-substituted benzophenones, uric and vilouric acids, tannic acid and its derivatives, hydroquinone and benzophenones. In accordance with this invention, an effective amount will normally be from about 0.01 to about 10% by weight, preferably from about 0.1 to about 5% by weight, based on the weight of the composition.

Typically, the active ingredient in deodorant-antiperspirant compositions is a basic aluminum compound. Examples of such materials are aluminum chlorhydroxide, basic aluminum bromide, iodide or nitrate, and basic aluminum hydroxy chloride-zirconyl hydroxy oxychloride.

Cleaning agents are typically anionic, cationic, non-ionic or amphoteric surfactants. Typical anionic surfactants are carboxylates, sulfonates, sulfates or phosphates, e.g. fatty acid soaps, salts of lauryl sulfate and salts of lauryl ether

sulfate. Examples of cationic surfactants are aliphatic mono, di and polyamines derived from fatty and rosin acids, amine oxides, ethoxylated alkyl amines and imidazolines. Examples of non-ionic surfactants are polyoxyethylene surfactants, alkylphenol ethoxylates, carboxylic acid esters, e.g., mono and diglycerides, polyoxyethylene esters and fatty acid diethanolamine condensates. Amphoteric surfactants are those containing combinations of the anionic and cationic groups described above, particularly those containing both acid carboxyls and basic nitrogen groups. Typical amphoteric surfactants are imidazolines and betaines, e.g., lauric and myristic imidazolines and betaines, and amidopropylbetaines.

A variety of medicinal agents also may be present as active ingredients in the compositions of the invention. Non-limiting examples are anti-acne additives, anti-cellulite agents, antihistamines, anti-inflammatory agents, antimicrobials, spermicides, antiseptics, antifungal agents and antiviral agents, and local anesthetics.

Active Ingredient Incorporation

As indicated previously, the cosmetic compositions of the present invention are generally prepared by adding the active cosmetic ingredients into the aqueous polyurethane dispersion.

For active cosmetic ingredients that are soluble in the aqueous vehicle of the dispersion, these can be added under standard mixing conditions so as to result in a composition that, after mixing (or remixing), has a substantially uniform composition.

For active cosmetic ingredients that are not soluble in the aqueous vehicle of the dispersion, it is preferably that the polyurethane ionomer act as a dispersing/emulsifying agent, the mixing should occur under high shear conditions known to those of ordinary skill in the relevant art. Alternatively, separate dispersing/emulsifying agents can be utilized.

For example, pigmented dispersions can be prepared using any conventional milling process known in the art. Most milling processes use a two-step process involving a first mixing step followed by a second grinding step. The first step comprises a mixing of all the ingredients, that is, pigment, aqueous dispersion and any optional additives to provide a blended "premix". Typically, any solid ingredients are added last. Mixing is generally done in a stirred mixing vessel, and high-speed dispersers (HSD) are particularly suitable for the mixing step. The second step comprises grinding of the premix to produce a pigmented dispersion. Preferably, grinding occurs by a media milling process, although other known milling techniques can also be used. Grinding can be accomplished by charging a media (such as zirconia) to the mill at a mill disc speed between 2000 rpm and 4000 rpm. The dispersion can be processed using a re-circulation grinding process, and flow rates though the mill are typically 200 to 500 grams/min. The milling may be done using a staged procedure. After completion of milling process, the dispersion is filled into a container, then typically further processed, for example, using conventional filtration procedures known in the art. The dispersions may be processed using ultrafiltration techniques that remove co-solvents and other contaminants, ions or impurities from the dispersion.

Typically, pigment levels (when pigments are used as the active cosmetic ingredient) are in the range of about 0.01 wt% to about 10 wt%, and more preferably from about 1 wt% to about 10 wt%, based on the weight of the cosmetic composition.

It is preferred that the resulting products are storage stable, which can be assessed after oven aging of samples for 1 week at 70°C. To the extent that the resulting products are not storage stable (separation of substantially uniform consistency of components), the consistency of the product should be readily restorable with simple mixing techniques (preferably low energy) such as container shaking.

Solidifying Agents

Solid/semi-sold compositions in accordance with the present invention are those considered non-flowable at ambient temperatures (25°C). Gels are generally included within the semi-solids classification. Examples of solid/semi-sold cosmetic compositions include lipsticks, antiperspirant sticks, stick mascara and eye liner, and the like.

The solids can be prepared by adding a solidifying to the aqueous dispersion of the polyurethane ionomer and cosmetic agent(s).

Solidifying agents include, for example, various waxes, gums and getting agents.

Cellulosic gums can be used as solidifying agents. For instance, US2003/0198616A1 describes a moisturizing skin gel wherein a water-soluble hydroxyalkylcellulose polymer typically performs a dual function of gelling the composition and forming a moisture barrier to reduce transepidermal water loss. Preferred cellulosic gums include water-soluble hydroxyalkylcellulose polymers such as hydroxymethylcellulose, hydroxyethylcellulose and hydroxypropylcellulose. Other thickening agents which have been used in skin-contacting compounds, include acacia, agar, alginate, carrageenan, gum tragacanth, xanthan gum, collagen, carboxypolymethylene, glyceryl monostearate, polyvinylpyrrolidone and polyacrylamide.

Other Ingredients

Fillers, plasticizers, silica sols, other polymer dispersions and the known leveling agents, wetting agents, antifoaming agents, stabilizers, preservatives and other additives known for the desired end use, may also be incorporated into the dispersions.

Surfactants may be used in the cosmetic compositions of the invention. Typical surfactants are disclosed in US2003/0007939A1.

Although the polyurethane ionomer is preferably dispersible in a continuous aqueous medium with out adding any emulsifier or surfactant, additional emulsifiers may be used in many preferred embodiments of the invention. Most emulsifiers approved for cosmetic use can be used. Operable emulsifiers include nonionic, anionic, cationic, amphoteric or zwitterionic and blends thereof. Suitable emulsifiers are disclosed in US3755560 and US4421769. Examples are polyethylene glycol 20, sorbitan monolaurate (Polysorbate 20), polyethylene glycol 20 stearyl ether (Brij 78, Steareth 20), polyethylene glycol ether of lauryl alcohol (Laureth 23), polysorbate 80 (Tween 80), and lecithin.

Other commonly used ingredients in cosmetic compositions include preservatives, which may be selected from the many that are known in the art and commercially available. Examples include benzyl alcohol, methyl paraben, propyl paraben, DMDM hydantoin, methylchloroisothiaoline, methylisothiazolinone, imidazolidinyl urea phenoxyethanol, sodium benzoate and benzoic acid. EDTA and salts thereof are often used to further enhance preservation.

Although additives such as those described above may be advantageously included in the compositions without limitation, the total amount of these additives generally ranges up to about 8.0 w%, and preferably up to about 3.0 wt%, based on the weight of the composition.

Well-known pharmacologically acceptable organic co-solvents may also be used in the cosmetic compositions of the present invention, although it is preferred that such compositions are substantially organic co-solvent free.

Product Forms

Cosmetic compositions based on the aqueous polyurethane dispersions are generally in the form of creams, emulsions, foams, gels, lotions, ointments, aerosols etc. The compositions thus may be in a wide variety of product types. These include but are not limited to solutions, lotions, emulsions, creams, gels, sticks, sprays, ointments, pastes, foams, mousses, shampoos, etc. Products employing these compositions include but are not restricted to skin care products, deodorants, anti-perspirants, insect repellants, anesthetics, shampoos, hair conditioners, sun care products, shower gels, soaps, hair styling gels, hair anti-dandruff compositions, hair growth promoter compositions, hair colorant compositions, hair bleaching agent compositions, hair anti-frizzing agent compositions, hair relaxer compositions, shaving product compositions, lubricating gel compositions, skin cleaning compositions, nail polish compositions and skin coloring compositions.

Most often cosmetic products contain an active ingredient incorporated in a delivery vehicle. The desired effect of a cosmetic product is achieved either by the cosmetically active ingredients or by the vehicle itself at the site of application, in most cases on the skin, nails or hair. With the aid of the vehicle, i.e. the vehicle acting as a carrier, the active ingredient is delivered to the application site where the desired effect is to be achieved.

The major types of personal care vehicles most frequently fall into the following categories: (a) solutions; (b) emulsions, both oil-in-water and water-in-oil; and including lotions and creams; (c) suspensions; (d) gels; and (e) solids and semi-solids including stick products. An extensive discussion of personal care and cosmetic vehicles is found in the previously incorporated Handbook of Cosmetic Science and Technology, Second Edition, edited by M Paye, A. O. Barel and H. I. Maibach, pages 99-123 (2005).

Gels

A "gel" in accordance with the present invention is a colloid in which the disperse phase has combined with the continuous phase to produce a viscous, jelly-like product.

Gels in accordance with the present invention can be aqueous or nonaqueous. The gels will typically comprise, in addition to the aqueous disperson of the polyurethane and cosmetic additive, a gelling agent such as described above. The vehicle of the gels will also typically comprise some solvent (preferably water).

Gels, in particular aqueous gels, are becoming increasingly popular in personal care products. Examples include, but are not restricted to, skin care products, cosmetics, tooth pastes, deodorants, anti-perspirants, insect repellants, anesthetics, shampoos, hair conditioners, sun care products, shower gels, soaps, hair styling gels, hair anti-dandruff compositions, hair growth promoter compositions, hair colorant compositions, hair bleaching agent compositions, hair anti-frizzing agent compositions, hair relaxer compositions, shaving product compositions, lubricating gel compositions, spermicidal gel compositions and skin cleaning compositions.

Emulsions

Emulsions are widely used as cosmetic products. By "emulsion" is meant a stable mixture of two or more immiscible liquids held in suspension by small percentages of substances called emulsifiers, which may be nonionic, anionic, cationic or zwitterionic. In the case of oil-in-water emulsions, the oil phase is the internal or dispersed phase, and the water phase is the external (continuous) or carrier phase.

If emulsions are liquid (flowable at ambient temperature), they are generally referred to as lotions. Creams are emulsions that occur in substantially non-flowable form (at ambient temperature). Generally creams do not flow through orifices under gravity because of their heavier consistency when compared to lotions. The consistency, or viscosity, of emulsions depends on several factors, including the ratio of internal to external phase, type of oil phase, and presence or absence of thickening agents in the continuous phase.

Two phase emulsion skin care preparations, such as lotions and creams, of the oil-in-water type are well-known in the cosmetic art and are useful in the subject invention. Triphase emulsion compositions, such as the water-in-oil-in-water type as disclosed in US4254105, are also useful in the subject invention. In general, such triphase emulsions contain water, emollients and emulsifiers as essential ingredients. Triple emulsion carrier systems comprising an oil-in-water-in-silicone fluid emulsion composition, as disclosed in US4960764, may also be useful in the subject invention.

Oils useful in emulsions, and also for co-solvents, include hydrocarbon oils and waxes (e.g., petrolatum, mineral oil, micro-crystalline waxes, polyalkenes, paraffins, cerasin, ozokerite, polyethylene, perhydrosqualene, poly alpha olefins, hydrogenated polyisobutenes and combinations thereof) and silicones (e.g., dimethicone copolyol, dimethylpolysiloxane, diethylpolysiloxane, mixed C1-C30 alkyl polysiloxanes, phenyl dimethicone, dimethiconol and combinations thereof). Preferred are non-volatile silicones selected from dimethicone, dimethiconol, mixed C1-C30 alkyl polysiloxane and combinations thereof; fatty acid derivatives, cholesterol, cholesterol derivatives, diglycerides and triglycerides (e.g., castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil, sesame oil, vegetable oils and vegetable oil derivatives, sunflower seed oil, coconut oil and derivatized coconut oil, cottonseed oil and derivatized cottonseed oil, jojoba oil, cocoa butter and combinations thereof, as well as any of the aforementioned oils that have been partially or fully hydrogenated), acetoglyceride esters (e.g., acetylated monoglycerides), alkyl esters, alkenyl esters (e.g., oleyl myristate, oleyl stearate, oleyl oleate, and combinations thereof), lanolin and its derivatives (e.g., lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol ricinoleate, hydroxylated lanolin, hydrogenated lanolin and combinations thereof), wax esters (e.g., beeswax and beeswax derivatives, spermaceti, myristyl myristate, stearyl stearate and combinations thereof), sterols and phospholipids, and combinations thereof. Examples of alkyl esters include isopropyl esters of fatty acids and long chain esters of long chain fatty acids, e.g., SEFA (sucrose esters of fatty acids), pentaerythritol esters, aromatic mono, di or triesters, cetyl ricinoleate, isopropyl palmitate, isopropyl myristate, cetyl ricinoleate and stearyl ricinoleate. Other examples include hexyl laurate, isohexyl laurate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof. Still other suitable oils include milk triglycerides (e.g., hydroxylated milk glyceride) and polyol fatty acid polyesters. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and combinations thereof.

Oil-in-water emulsions are widely used in cosmetic products, preferably as skin care products, skin moisturizers, cosmetics, deodorants, anti-perspirants, insect repellants, anesthetics, medicinal agents, hair conditioners, sun care products, soaps, hair anti-dandruff compositions, hair growth promoter compositions, hair colorant compositions, hair bleaching agent compositions, hair anti-frizzing agent compositions, hair relaxer compositions and shaving product compositions.

Suspensions

Still another aspect of the invention is a personal care composition comprising a suspension. Suspensions consist of solid particles dispersed in a liquid or semi-solid medium. Sedimentation during storage is minimized by reducing particle size and/or by increasing the viscosity of the carrier phase. Typical uses of suspensions are essentially the same as those listed above for oil-in-water emulsions.

(Semi)Solids (Other than Gels)

Solid delivery vehicles are generally cast in an elongated form as sticks. By rubbing the sticks onto the skin a variety of personal care ingredients can be delivered. Examples are lipsticks and antiperspirant/deodorant sticks. There are several ways of achieving solid stick properties, such as mixtures of waxes, pigments and oils; solutions based on

aqueous, propylene glycol and/or alcohol mixtures solidified usually by sodium stearate; and matrices consisting of a high-boiling silicone gelled by fatty alcohol (e.g. stearyl alcohol). Preferably, the solidifying agent is selected from the group consisting of a wax and sodium stearate.

Solid stick formulations are most frequently used in lipsticks, antiperspirants/deodorants, skin moisturizers, cosmetics, insect repellants, sun care products, soaps and other skin cleaning compositions, skin sanitizers, shaving product compositions, skin cleaning compositions and anesthetics.

## Other Forms

It should be noted that most of the liquid vehicles described above can be in the form of foams, which are dispersions of gas in the liquid phase. The gas globules may be of any size, from colloidal to macroscopic, as in soap bubbles. Typical liquid foams are those used in shaving creams, etc.

Liquid or solid vehicle systems can also be applied as aerosols. By "aerosol" is meant a suspension of liquid or solid particles in a gas, the particles often being in the colloidal size range. Included are fine sprays (perfumes, insecticides, inhalants, anti-perspirants, etc.). Suspensions of various kinds are prepared by placing the components, together with a compressed gas, in a container (bomb). The pressure of the gas causes the mixture to be released as a fine spray (aerosol) when a valve is opened. Examples are perfumes, deodorants, shaving cream, and the like. The propellant gas may be, for example, a hydrocarbon (propane, isobutene), a chlorofluorocarbon, carbon dioxide or nitrous oxide.

## Examples

Number-average molecular weights (Mn) were determined by end-group analysis using NMR spectroscopic methods.

Polydispersity (Mw/Mn) was measured by GPC.

Color was measured as APHA values (Platinum-Cobalt System) according to ASTM D-1209.

All percentages, parts, etc., are by weight unless otherwise indicated.

## Example 1

This example illustrates preparation of a substantially organic solvent-free polyurethane dispersion from polytrimethylene ether glycol, isophorone diisocyanate and dimethylolpropionic acid ionic reactant, which was chain extended after inversion with a combination of diamine and polyamine.

A 2L reactor was loaded with 201.11 g PO3G (Mn of 2000) and heated to 100°C under vacuum until the contents had less than 500 ppm water. The reactor was cooled to 40°C, and acetone (99 g) and 0.13 g dibutyltin dilaurate catalyst were added. 53.01 g isophorone diisocyanate was added over 1 hr, and rinsed in with 2.6 g dry acetone. The reaction was allowed to continue at 50°C for 2.5 hr, and then the wt% NCO was determined to be below 3.5%. Dimethylol proprionic acid (12.98 g) and triethyl amine (8.82 g) were added, followed by a rinse with dry acetone (3.16 g). The reaction was held at 50°C for 2 hrs, and the wt% NCO was determined to be below 0.6%. The resulting polyurethane solution was inverted under high speed mixing while adding 575 g water immediately followed by ethylene diamine (7.52 g) and triethylene tetraamine (36.6 g). The acetone was distilled off under reduced pressure at 70°C.

The resulting P03G-based polyurethane dispersion had a viscosity of 13.4 cPs, 30.2 wt% solids, a titrated acid number of 17.6 mg KOH/ g solids, and an average particle size of 37 nm with 95% below 63 nm.

## Example 2 - Flowable Lipstick/Lip Gloss Formulation

| | |
|---|---|
| Polyurethane dispersion | 95 g |
| Pigment | 1-2 g |
| Glycerol | 1-3 g |

## Example 3 - Flowable Lipstick/Lip Gloss Formulation

| | |
|---|---|
| Polyurethane dispersion | 90 to 98 wt% |

(continued)

| | |
|---|---|
| Bordeaux mica pigment | 1 to 5 wt% |
| 1,3-propane diol | 1 to 5 wt% (preferably biologically derived) |

Example 4 - Flowable Hair Care Formulation

| | |
|---|---|
| Polyurethane dispersion | 5 to 20 wt% |
| Diethyl ether | 25 to 35 wt% |
| Ethanol | 0 to 25 wt% |
| Deionized water | 30 to 60 wt% |

**Claims**

1. A cosmetic composition comprising:

   (i) an aqueous continuous phase having dispersed therein water-dispersible polyurethane particles of less than 1 μm and at least one cosmetic additive, or
   (ii) a solid or semi-solid continuous phase having dispersed therein an aqueous vehicle, water-dispersible polyurethane particles of less than 1 μm and at least one cosmetic additive,

   wherein the water-dispersible polyurethane is a polyurethane ionomer comprising a polymeric backbone having ionic and/or ionizable functionality incorporated into, pendant from and/or terminating said polymeric backbone, wherein the polymeric backbone comprises one or more non-ionic segments derived from a reaction product of polytrimethylene ether glycol and a polyisocyanate.

2. The cosmetic composition of claim 1, comprising an aqueous continuous phase having dispersed therein water-dispersible polyurethane particles of less than 1 μm and at least one cosmetic additive.

3. The cosmetic composition of claim 2, wherein the polyurethane ionomer has sufficient ionic functionality in order to render the polyurethane dispersible in the aqueous continuous phase.

4. The cosmetic composition of claim 2 or 3, which is an oil-in-water emulsion, a suspension or a gel.

5. The cosmetic composition of claim 1, comprising a solid or semi-solid continuous phase having dispersed therein an aqueous vehicle, water-dispersible polyurethane particles of less than 1 μm and at least one cosmetic additive.

6. The cosmetic composition of any of claims 1-5, wherein at least 20 wt % of the polyurethane comprises one or more non-ionic segments of the general formula (I):

   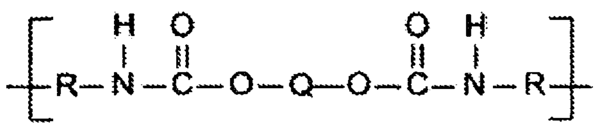

   (I)

   wherein:

   each R individually is the residue of a diisocyanate compound after abstraction of the isocyanate groups; and
   Q is the residue of an oligomeric or polymeric diol after abstraction of the hydroxyl groups, wherein the oligomeric or polymeric diol is polytrimethylene ether glycol.

**7.** The cosmetic composition of any of claims 1-6, wherein the polyurethane is obtainable from (a) a polyol component comprising at least 40 wt % polytrimethylene ether glycol, based on the weight of the polyol component; (b) a polyisocyanate component comprising a diisocyanate; and (c) a hydrophilic reactant comprising a compound selected from the group consisting of (i) mono or diisocyanate containing an ionic and/or ionizable group, and (ii) an isocyanate reactive ingredient containing an ionic and/or ionizable group.

**8.** The cosmetic composition of claim 7, wherein the polyol component of the polyurethane comprises at least 90 wt % polytrimethylene ether glycol.

**9.** The cosmetic composition of any of claims 1-8, wherein the polytrimethylene ether glycol comprises trimethylene ether units from 1,3-propane diol having the following characteristics:

(1) an ultraviolet absorption at 220 nm of less than 0.200, and at 250 nm of less than 0.075, and at 275 nm of less than 0.075; and/or
(2) a composition having L*a*b* 'b*' color value of less than 0.15 (ASTM D6290), and an absorbance at 270 nm of less than 0.075; and/or
(3) a peroxide composition of less than 10 ppm; and/or
(4) a concentration of total organic impurities (organic compounds other than 1,3-propanediol) of less than 400 ppm, as measured by gas chromatography.

**10.** The cosmetic composition of any of claims 1-9, wherein the polytrimethylene ether glycol comprises trimethylene ether units from biologically-derived 1,3-propane diol.

**11.** The cosmetic composition of any of claims 1-9, wherein the cosmetic additive is a perfume, fragrance, skin coolant, emollient, deodorant, antiperspirant agent, moisturizing agent, hair treatment agent, beauty aid, medicinal agent, humectant, sunscreen, or cleaning agent.

**12.** A process for preparing a cosmetic composition comprising the steps of:

mixing an active cosmetic ingredient into an aqueous dispersion of water-dispersible polyurethane particles of less than 1 μm comprising an aqueous phase and a water-dispersible polyurethane ionomer, wherein the water-dispersible polyurethane ionomer comprises a polymeric backbone having ionic and/or ionizable functionality incorporated into, pendant from and/or terminating said polymeric backbone, wherein the polymeric backbone comprises one or more non-ionic segments derived from a reaction product of polytrimethylene ether glycol and a polyisocyanate, and wherein said polyurethane ionomer has sufficient ionic functionality in order to render the polyurethane dispersible in the aqueous continuous phase,

wherein, if the cosmetic composition is a solid or semi-solid, the process comprises the further step of:

adding a solidifying agent to the aqueous dispersion to result in a solid or semi-sold product.

**13.** The process of claim 12, wherein the cosmetic composition is as set forth in any of claims 1-10.

## Patentansprüche

**1.** Kosmetische Zusammensetzung umfassend:

(i) eine wässrige kontinuierliche Phase, in der wasserdispergierbare Polyurethanteilchen von weniger als 1 μm und mindestens ein kosmetisches Zusatzmittel dispergiert sind, oder
(ii) eine feste oder halbfeste kontinuierliche Phase, in der ein wässriges Vehikel, wasserdispergierbare Polyurethanteilchen von weniger als 1 μm und mindestens ein kosmetisches Zusatzmittel dispergiert sind,

wobei das wasserdispergierbare Polyurethan ein Polyurethanionomer ist, das eine polymere Rückgratkette umfasst, wobei eine ionische und/oder ionisierbare Funktionalität in die polymere Rückgratkette eingearbeitet ist, eine Seitengruppe davon bildet und/oder diese abschließt, wobei die polymere Rückgratkette ein oder mehrere nichtionische Segmente umfasst, die von einem Reaktionsprodukt von Polytrimethylenetherglykol und einem Polyisocyanat abgeleitet sind.

2. Kosmetische Zusammensetzung nach Anspruch 1, die eine wässrige kontinuierliche Phase umfasst, in der wasserdispergierbare Polyurethanteilchen von weniger als 1 μm und mindestens ein kosmetisches Zusatzmittel dispergiert sind.

3. Kosmetische Zusammensetzung nach Anspruch 2, wobei das Polyurethanionomer eine ausreichende ionische Funktionalität aufweist, um das Polyurethan in der wässrigen kontinuierlichen Phase dispergierbar zu machen.

4. Kosmetische Zusammensetzung nach Anspruch 2 oder 3, die eine Öl-in-Wasser-Emulsion, eine Suspension oder ein Gel ist.

5. Kosmetische Zusammensetzung nach Anspruch 1, umfassend eine feste oder halbfeste kontinuierliche Phase, in der ein wässriges Vehikel, wasserdispergierbare Polyurethanteilchen von weniger als 1 μm und mindestens ein kosmetisches Zusatzmittel dispergiert sind.

6. Kosmetische Zusammensetzung nach einem der Ansprüche 1-5, wobei mindestens 20 Gew.-% des Polyurethans ein oder mehrere nichtionische Segmente der allgemeinen Formel (I):

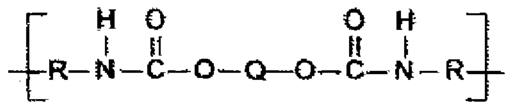

(I)

umfassen, wobei
jedes R einzeln der Rest einer Diisocyanatverbindung nach Abtrennung der Isocyanatgruppen ist; und
Q der Rest eines oligomeren oder polymeren Diols nach Abtrennen der Hydroxylgruppen ist, wobei das oligomere oder polymere Diols Polytrimethylenetherglykol ist.

7. Kosmetische Zusammensetzung nach einem der Ansprüche 1-6, wobei das Polyurethan erhältlich ist von (a) einer Polyolkomponente, die mindestens 40 Gew.-% Polytrimethylenetherglykol, auf das Gewicht der Polyolkomponente bezogen, umfasst; (b) einer Polyisocyanatkomponente, die ein Diisocyanat umfasst; und (c) einem hydrophilen Reaktanden, der eine Verbindung umfasst ausgewählt aus der Gruppe bestehend aus (i) Mono- oder Düsocyanat, das eine ionische und/oder ionisierbare Gruppe enthält und (ii) einem isocyanatreaktiven Bestandteil, der eine ionische und/oder ionisierbare Gruppe enthält.

8. Kosmetische Zusammensetzung nach Anspruch 7, wobei die Polyolkomponente des Polyurethans mindestens 90 Gew.-% Polytrimethylenetherglykol umfasst.

9. Kosmetische Zusammensetzung nach einem der Ansprüche 1-8, wobei das Polytrimethylenetherglykol Trimethylenethereinheiten von 1,3-Propandiol umfasst, die die folgenden charakteristischen Eigenschaften aufweisen:

(1) eine Ultraviolettabsorption bei 220 nm von weniger als 0,200 und bei 250 nm von weniger als 0,075 und bei 275 nm von weniger als 0,075; und/oder
(2) eine Zusammensetzung, die einen L*a*b*'b*'-Farbwert von weniger als 0,15 (ASTM D6290) und eine Extinktion bei 270 nm von weniger als 0,075 aufweist; und/oder
(3) eine Peroxidzusammensetzung von weniger als 10 ppm; und/oder
(4) einer Konzentration von gesamten organischen Verunreinigungen (organischen Verbindungen, bei denen es sich nicht um 1,3-Propandiol handelt) von weniger als 400 ppm, wie durch Gaschromatographie gemessen.

10. Kosmetische Zusammensetzung nach einem der Ansprüche 1-9, wobei das Polytrimethylenetherglykol Trimethylenethereinheiten von biologisch abgeleitetem 1,3-Propandiol umfasst.

11. Kosmetische Zusammensetzung nach einem der Ansprüche 1-9, wobei das kosmetische Zusatzmittel ein Parfüm, Duftstoff, Hautkühlmittel, Emolliens, Deodorant, schweißhemmendes Mittel, Feuchtigkeitsmittel, Haarbehandlungsmittel, Schönheitsmittel, medizinisches Mittel, Feuchthaltemittel, Sonnenschutzmittel oder Reinigungsmittel ist.

12. Verfahren zum Herstellen einer kosmetischen Zusammensetzung, umfassend die Schritte des:

Mischens eines aktiven kosmetischen Bestandteils in eine wässrige Dispersion von wasserdispergierbaren Polyurethanteilchen von weniger als 1 μm, umfassend eine wässrige Phase und ein wasserdispergierbares Polyurethanionomer, wobei das wasserdispergierbare Polyurethanionomer eine polymere Rückgratkette umfasst, die eine ionische und/oder ionisierbare Funktionalität aufweist, die in die polymere Rückgratkette eingearbeitet ist, eine Seitengruppe davon bildet und/oder diese abschließt, wobei die polymere Rückgratkette ein oder mehrere nichtionische Segmente umfasst, die von einem Reaktionsprodukt von Polytrimethylenetherglykol und einem Polyisocyanat abgeleitet sind, und wobei das Polyurethanionomer eine ausreichende ionische Funktionalität aufweist, um das Polyurethan in der wässrigen kontinuierlichen Phase dispergierbar zu machen

wobei, wenn die kosmetische Zusammensetzung ein Feststoff oder Halbfeststoff ist, das Verfahren die weiteren Schritte umfasst des:

Hinzugebens eines Verfestigungsmittels zu der wässrigen Dispersion, um zu einem festen oder halbfesten Produkt zu führen.

**13.** Verfahren nach Anspruch 12, wobei die kosmetische Zusammensetzung wie in einem der Ansprüche 1-10 aufgeführt ist.

## Revendications

**1.** Composition cosmétique comprenant:

(i) une phase continue aqueuse ayant dispersées à l'intérieur des particules de polyuréthane pouvant être dispersées dans l'eau inférieures à 1 μm et au moins un additif pour cosmétique, ou
(ii) une phase continue solide ou semi-solide ayant dispersés à l'intérieur un véhicule aqueux, des particules de polyuréthane pouvant être dispersées dans l'eau inférieures à 1 μm et au moins un additif pour cosmétique,

dans laquelle le polyuréthane pouvant être dispersé dans l'eau est un ionomère de polyuréthane comprenant un squelette polymère ayant une fonctionnalité ionique et/ou ionisable incorporée dedans, accrochée sur et/ou achevant ledit squelette polymère, dans laquelle le squelette polymère comprend un ou plusieurs segment(s) non ionique(s) dérivé(s) d'un produit réactionnel de poly(éther de triméthylène) glycol et d'un polyisocyanate.

**2.** Composition cosmétique selon la revendication 1, comprenant une phase continue aqueuse ayant dispersées à l'intérieur des particules de polyuréthane pouvant être dispersées dans l'eau inférieures à 1 μm et au moins un additif pour cosmétique.

**3.** Composition cosmétique selon la revendication 2, dans laquelle le ionomère de polyuréthane a une fonctionnalité ionique suffisante pour rendre le polyuréthane dispersible dans la phase continue aqueuse.

**4.** Composition cosmétique selon la revendication 2 ou 3, qui est une émulsion huile-dans-eau, une suspension ou un gel.

**5.** Composition cosmétique selon la revendication 1, comprenant une phase continue solide ou semi-solide ayant dispersés à l'intérieur un véhicule aqueux, des particules de polyuréthane pouvant être dispersées dans l'eau inférieures à 1 μm et au moins un additif pour cosmétique.

**6.** Composition cosmétique selon l'une quelconque des revendications 1 à 5, dans laquelle au moins 20 % en pds du polyuréthane comprend un ou plusieurs segment(s) non ionique(s) de la formule générale (I):

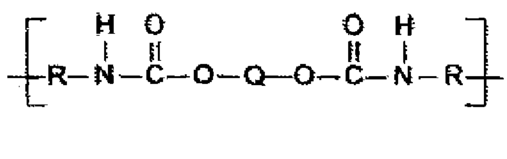

(I)

dans laquelle:

chaque R individuellement est le résidu d'un composé diisocyanate après abstraction des groupes isocyanates; et

Q est le résidu d'un diol oligomère ou polymère après abstraction des groupes hydroxyle, dans lequel le diol oligomère ou polymère est le poly(éther de triméthylène) glycol.

**7.** Composition cosmétique selon l'une quelconque des revendications 1 à 6, dans laquelle le polyuréthane peut être obtenu à partir de (a) un composant polyol comprenant au moins 40 % en pds de poly(éther de triméthylène) glycol, basé sur le poids du composant polyol; (b) un composant polyisocyanate comprenant un diisocyanate; et (c) un réactif hydrophile comprenant un composé choisi parmi le groupe constitué de (i) mono ou diisocyanate contenant un groupe ionique et/ou ionisable, et (ii) d'un ingrédient réactif isocyanate contenant un groupe ionique et/ou ionisable.

**8.** Composition cosmétique selon la revendication 7, dans laquelle le composant polyol du polyuréthane comprend au moins 90 % en pds de poly(éther de triméthylène) glycol.

**9.** Composition cosmétique selon l'une quelconque des revendications 1 à 8, dans laquelle le poly(éther de triméthylène) glycol comprend des motifs éther de triméthylène de 1,3-propane diol ayant les caractéristiques suivantes:

(1) une absorption dans la gamme de l'ultraviolet à 220 nm inférieure à 0,200, et à 250 nm inférieure à 0,075, et à 275 nm inférieure à 0,075; et/ou

(2) une composition ayant une valeur de couleur « b* » L*a*b* inférieure à 0,15 (ASTM D6290), et une absorbance à 270 nm inférieure à 0,075; et/ou

(3) une composition peroxyde inférieure à 10 ppm; et/ou

(4) une concentration des impuretés organiques totales (composés organiques autres que le 1,3-propanediol) inférieure à 400 ppm, telle que mesurée par chromatographie en phase gazeuse.

**10.** Composition cosmétique selon l'une quelconque des revendications 1 à 9, dans laquelle le poly(éther de triméthylène) glycol comprend des motifs éther de triméthylène provenant de 1,3-propane diol d'origine biologique.

**11.** Composition cosmétique selon l'une quelconque des revendications 1 à 9, dans laquelle l'additif pour cosmétique est un parfum, une fragrance, un rafraîchissant cutané, un émollient, un déodorant, un agent antiperspirant, un agent hydratant, un agent de traitement capillaire, un auxiliaire de beauté, un agent médicinal, un humectant, un écran solaire, ou un agent de nettoyage.

**12.** Procédé de préparation d'une composition cosmétique comprenant les étapes de:

mélange d'un ingrédient cosmétique actif dans une dispersion aqueuse de particules de polyuréthane pouvant être dispersées dans l'eau inférieures à 1 μm comprenant une phase aqueuse et un ionomère de polyuréthane pouvant être dispersé dans l'eau, dans lequel l'ionomère de polyuréthane pouvant être dispersé dans l'eau comprend un squelette polymère ayant une fonctionnalité ionique et/ou ionisable incorporée dans, accrochée à et/ou terminant ledit squelette polymère, dans lequel le squelette polymère comprend un ou plusieurs segment (s) non ionique(s) dérivé(s) d'un produit réactionnel de poly(éther de triméthylène) glycol et un polyisocyanate, et dans lequel ledit ionomère de polyuréthane a une fonctionnalité ionique suffisante pour rendre le polyuréthane dispersible dans la phase continue aqueuse,

dans lequel, si la composition cosmétique est un solide ou un semi-solide, le procédé comprend l'étape supplémentaire de:

l'addition d'un agent de solidification à la dispersion aqueuse pour résulter en un produit solide ou semi-solide.

**13.** Procédé selon la revendication 12, dans lequel la composition cosmétique est telle qu'exposée dans l'une quelconque des revendications 1 à 10.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5874072 A
- US 5968494 A
- US 5972354 A
- US 20020034558 A1
- US 20040141942 A1
- US 78209807 A
- US 5633362 A
- US 5686276 A
- US 5821092 A
- US 20040260125 A1
- US 20040225161 A1
- US 20050069997 A1
- US 20050020805 A1
- US 6977291 B
- US 6720459 B
- US 20040030095 A1
- US 6608168 B
- US 6316586 B
- US 4701480 A
- US 6248839 B
- US 5990245 A
- US 4647643 A
- US 5760123 A
- US 6046295 A
- US 3479310 A
- US 4303774 A
- US 4108814 A
- US 4408008 A
- US 3419533 A
- US 3412054 A
- US 5173526 A
- US 4644030 A
- US 5488383 A
- US 5569705 A
- US 4269748 A
- US 4829122 A
- US 4501852 A
- US 20050215663 A1
- US 20030198616 A1
- US 20030007939 A1
- US 3755560 A
- US 4421769 A
- US 4254105 A
- US 4960764 A


### Non-patent literature cited in the description


- Handbook of Cosmetic Science and Technology. 2005, 99-123
- Source Apportionment of Atmospheric Particles. **Currie, L. A.** Characterization of Environmental Particles. Lewis Publishers, Inc, 1992, vol. I, 3-74
- **Hsieh, Y.** *Soil Sci. Soc. Am J.,* 1992, vol. 56, 460
- **Weber et al.** *J. Agric. Food Chem.,* 1997, vol. 45, 2942